# FASCICULE DE BREVET EUROPEEN

(11) **EP 1 046 627 B1**
(45) Date de publication et mention de la délivrance du brevet: **11.02.2004**
(21) Numéro de dépôt: 00201285.4
(22) Date de dépôt: 10.04.2000
(51) Int. Cl.: C07B 57/00, C07C 275/24, C07C 227/30, C07C 271/54, C07C 333/04

(54) **Procédé pour la séparation d'énantiomères et réactif énantiopur**
Enantiomerisch reine Reagenzien und Verfahren zur Trennung von Enantiomeren
Enantiopure reagents and process for the separation of enantiomers

(30) Priorité: 21.04.1999 BE 9900280
(43) Date de publication de la demande: 25.10.2000
(73) Titulaire: SOLVAY (Société Anonyme), 1050 Bruxelles (BE)
(72) Inventeur: Delplanche, Thierry, 1348 Ottignies-Louvain-la-Neuve (BE); Callens, Roland, 1850 Grimbergen (BE)
(74) Mandataire: Jacques, Philippe

(56) Documents cités:
- GB-A- 2 127 020
- K. IWAKI: "Activated carbamate reagent as chiral derivatizing agent for liquid chromatographic optical resolution of enantiomeric amino compounds" CHROMATOGRAPHIA, vol. 23, no. 12, décembre 1987 (1987-12), pages 899-902, XP000865626 Wiesbaden
- J. MAIBAUM: "Indirect high-performance liquid chromatographic resolution of racemic tertiary amines as their diastereomeric urea derivatives after N-dealkylation" JOURNAL OF CHROMATOGRAPHY, vol. 436, 1988, pages 269-278, XP000857562 AMSTERDAM NL
- C. J. GRAY: "Preparation and properties of some alpha-aza-amino-acid derivatives, their possible use in peptide synthesis" TETRAHEDRON, (INCL TETRAHEDRON REPORTS), vol. 33, 1977, pages 739-743, XP002126421 OXFORD GB
- C. J. GRAY: "Synthesis and spectroscopic properties of azaglutamine amino acid and peptide deivatives" SYNTHESIS, février 1991 (1991-02), pages 141-146, XP002126422 STUTTGART DE
- CHEMICAL ABSTRACTS, vol. 102, no. 19, 13 mai 1985 (1985-05-13) Columbus, Ohio, US; abstract no. 167132f, P. MARFEY: "Determination of D-amino acids. II. Use of a bifunctional reagent, 1,5-difluoro-2,4-dinitrobenzene." page 659; colonne 1; XP002126423 & CARLSBERG RES. COMMUN., vol. 49, no. 6, 1984, pages 591-596,

## Description

L'invention se rapporte à un procédé pour la séparation d'énantiomères et à des réactifs à base d'un aminoacide énantiopur, utilisables en séparation d'énantiomères.

La séparation d'énantiomères est une question de grande importance en industrie pharmaceutique, chimique et biotechnologique. En effet les deux énantiomères d'une substance chimique de constitution identique peuvent avoir des activités biologiques radicalement différentes. Il est ainsi souhaitable de disposer de réactifs et de techniques de séparation permettant de séparer les énantiomères et d'analyser la pureté énantiomérique des produits pharmaceutiques, chimiques et biotechnologiques.

Un article de Marfey, P., (Carlsberg Res. Comm. 49, 1984, 591-596) décrit un procédé de séparation d'énantiomères par RP-HPLC. Selon ce procédé connu, on met en oeuvre du 1-Fluoro-2,4-dinitrophényl-5-L-Alaninamide à titre de réactif pour la dérivatisation d'aminoacides. D'autres procédés similaires sont également connus. Le procédé et réactif décrits par Marfey ainsi que les autres procédés et réactifs présentent toutefois de nombreux inconvénients. Iwaki et al. Chromatographia, 23, N°12 (1987) p. 899-902, décrit la séparation de composés énantiométriques aminés par dérivatisation avec des succinimido carbamates de phényléthylamine ou de naphtyléthylamine, suivie de chromatographie HPLC.

Le dérivé obtenu dans la réaction de l'aminoacide avec le réactif doit être isolé par des opérations successives de neutralisation, de séchage, de redissolution et de filtration. Ces opérations demandent beaucoup de temps et sont donc peu intéressantes pour une application industrielle. De plus on risque, dans des cas d'applications analytiques, des erreurs dans les résultats d'analyse, causées par une solubilité différente des dérivés diastéréomériques dans le solvant de redissolution. Lorsqu'on effectue des analyses quantitatives à l'aide de la spectrométrie UV, on rencontre, dans le procédé connu, des difficultés dues à des différences de coefficient d'absorption des dérivés diastéréomériques. Enfin, le prix élévé du réactif rend souhaitable de trouver des alternatives.

L'invention vise à remédier à ces problèmes.

L'invention concerne dès lors un procédé pour la séparation d'énantiomères comprenant au moins un groupement fonctionnel libre dans lequel
(a) on fait réagir un mélange comprenant les énantiomères en milieu basique avec un réactif à base d'un aminoacide énantiopur, réactif dans lequel au moins un groupement amino de l'aminoacide porte un groupement activant pour former un précurseur actif d'un groupement isocyanate et dans lequel au moins un groupement carboxyle de l'aminoacide est substitué et
(b) on soumet le mélange de diastéréomères obtenu à une opération de séparation.

Il a été trouvé, de manière surprenante, que le procédé selon l'invention permet d'obtenir de bons résultats en séparation d'énantiomères comprenant au moins un groupement fonctionnel libre, notamment dans des applications analytiques quantitatives. Le procédé selon l'invention permet une dérivatisation et séparation d'énantiomères, rapides et sous des conditions souples et économiques.

L'invention concerne aussi un réactif à base d'un aminoacide énantiopur dans lequel au moins un groupement amino de l'aminoacide porte un groupement activant pour former un précurseur actif d'un groupement isocyanate et dans lequel au moins un groupement carboxyle de l'aminoacide est substitué.

Par aminoacide on entend désigner, aux fins de la présente invention tout composé comprenant au moins un groupement NH₂ et au moins un groupement carboxyle. Les aminoacides utilisés dans la présente invention sont des aminoacides chiraux contenant au moins un carbone asymétrique. On peut mettre en oeuvre tout aminoacide chiral bien connu en lui-même, d'origine naturelle ou synthétique.

Des exemples de réactifs selon l'invention sont à base, par exemple, des aminoacides naturels suivants :
Alanine, Valine, Norvaline, Leucine, Norleucine, Isoleucine, Sérine, Isosérine, Homosérine, Thréonine, Allothréonine, Méthionine, Ethionine, Acide Glutamique, Acide Aspartique, Asparagine, Cystéine, Cystine, Phénylalanine, Tyrosine, Tryptophane, Lysine, Arginine, Histidine, Omithine, Glutamine et Citrulline.

Les énantiomères non naturels sont également utilisables.

Des exemples d'aminoacides d'origine synthétique utilisables comme base du réactif selon l'invention comprennent, par exemple, les aminoacides suivants : (1-Naphtyl)alanine, (2-Naphtyl)alanine, Homophénylalanine, (4-Chlorophényl)alanine, (4-Fluorophényl)alanine, (3-Pyridyl)alanine, Phénylglycine, Acide Diaminopimélique (Acide 2-6-Diaminoheptane-1,7-dïoique), Acide 2-aminobutyrique, Acide-2-aminotétraline-2-carboxylique, Erythro β-Méthylphenylalanine, Threo β-Méthylphenylalanine, (2-Méthoxyphenyl)alanine, Acide 1-amino-5-hydroxyindane-2-carboxylique, Acide 2-Aminoheptane-1,7-dïoique, (2,6-Diméthyl-4-hydroxyphényl)alanine, Erythro β-Méthyltyrosine, Threo β-Méthyltyrosine.

Par aminoacide énantiopur on entend désigner un aminoacide chiral constitué essentiellement d'un énantiomère. L'excès énantiomérique (ee) est défini : ee(%) = 100(x₁-x₂)/(x₁+x₂) avec x₁>x₂; x₁ et x₂ représentent la teneur du mélange en énantiomère 1 ou 2 respectivement.

On met généralement en oeuvre un aminoacide énantiopur dont l'excès énantiomérique est supérieur ou égal à 99 %. On préfère un aminoacide énantiopur dont l'excès énantiomérique est supérieur ou égal à 99,5 %. De manière particulièrement préférée on met en oeuvre un aminoacide énantiopur dont l'excès énantiomérique est supérieur ou égal à 99,9 % .

Tout aminoacide énantiopur est utilisable comme base du réactif selon l'invention. De préférence l'aminoacide énantiopur est sélectionné parmi les aminoacides d'origine naturelle ou synthétique nommés ci-dessus. Les aminoacides comprenant au moins un noyau aromatique tels que par exemple la phénylalanine ou ses dérivés conviennent bien à titre d'aminoacide énantiopur. De façon particulièrement préférée, l'aminoacide énantiopur est sélectionné parmi la phénylalanine, la (1-naphtyl)alanine, la (2-naphtyl)alanine ou le α ou β tryptophane ((2-indolyl)alanine ou (3-indolyl)alanine), éventuellement substitués.

Dans le réactif selon l'invention, au moins un groupement amino de l'aminoacide énantiopur porte un groupement activant pour former un précurseur actif d'un groupement isocyanate.

Par précurseur actif d'un groupement isocyanate, on entend désigner tout précurseur qui lorsqu'il est mis en oeuvre dans un solvant utilisable dans le procédé selon l'invention avec un équivalent de phénylalanine en présence de 1 équivalent de base réagit à une température inférieure ou égale à 35 °C en une durée inférieure ou égale à 30 min essentiellement complètement, pour former l'urée correspondant. De préférence le précurseur réactif libère le groupement isocyanate à une température inférieure ou égale à 30 °C en une durée inférieure ou égale à 15 min. De manière tout particulièrement préférée, le précurseur réactif libère le groupement isocyanate à température ambiante en une durée inférieure ou égale à 10 min. Des conditions de test utilisables pour déterminer le précurseur actif sont décrites, par exemple dans l'exemple 3 plus bas.

Le groupement activant est généralement constitué d'un dérivé carbonyle lié à un substituant électronégatif. On peut par exemple utiliser à titre de groupement activant un groupement aryloxycarbonyl, hétéroaryloxycarbonyl, 1,3-imidazolyl-N-carbonyl ou 1,2,4-triazolyl-N-carbonyl. Parmi les groupes aryloxycarbonyl conviennent bien ceux qui portent au moins un substituant -I, - M sur un noyau aromatique. Un substituant -I, -M est un groupement qui a un effet négatif inductif et de résonance comme défini en J. March, Advanced Organic Chemistry, 4. Ed., 1992, p. 17-19, 273-275. Parmi les substituants -I, - M figurent, par exemple -NO₂, -SO₂R, -SO₂OR, -NR₃⁺, SR₂⁺. Les substituants se trouvent de préférence à au moins une des positions 2, 4 ou 6 du noyau aromatique ou à des positions analogues aux positions 2 ou 4 dans des systèmes aromatiques condensés. On préfère utiliser un groupement activant aryloxycarbonyl qui porte au moins un substituant nitro sur un noyau aromatique. Le groupement (4-nitrophenyloxy)carbonyl est particulièrement préféré.

Dans une variante, le substituant electronégatif comprend un substituant -I (effet négatif inductif) indépendemment de l'effet de resonance (M) tels que définis plus haut. Le groupement activant est dans cette variante, de préférence, un groupement aryloxycarbonyl porteur d'au moins un substituant -I. Le substituant -I se trouve, de préférence aux positions renseignées plus haut pour les substituants -I, -M. Des exemples de substituants -I utilisables dans le réactif selon l'invention sont, par exemple, les halogènes. Le chlore et le fluor conviennent bien. Le fluor est préféré.

Dans une variante de l'invention, toutes autres choses restant égales, le réactif est à base d'un aminoacide énantiopur dans lequel au moins un groupement amino de l'aminoacide porte un groupement activant pour former un précurseur actif d'un groupement isothiocyanate. On peut par exemple utiliser à titre de groupement activant un groupement thiocarbonyl lié à un groupement electronégatif tel que décrit plus haut. Un groupement (4-nitrophényloxy)thiocarbonyl ou (4-fluorophényloxy)thiocarbonyl est préféré.

Il a été trouvé que dans cette variante de l'invention la réaction du réactif avec le composé organique comprenant un groupement fonctionnel libre donne généralement lieu à la formation de dérivés comprenant un groupement thiocarbonyl ce qui peut donner une séparation d'énantiomères encore améliorée par rapport aux dérivés oxygénés. La présence de soufre dans les dérivés diastéréomériques facilite encore la détection desdites dérivés, notamment par spéctrométrie UV.

Dans le réactif selon l'invention, au moins un groupement carboxyle de l'aminoacide est substitué.

Généralement, le substituant par lequel le groupement carboxyle de l'aminoacide est substitué ne comprend pas de groupement fonctionnel libre, susceptible de réagir avec le précurseur actif. Parmi les substituants utilisables figurent par exemple, les groupements alkyles comprenant de 1 à 4 atomes, linéaires ou ramifiés tels que le groupement méthyl, éthyl, n-propyl, i-propyl, n-butyl, i-butyl ou t-butyl, les éthers, les groupements aryle, les groupements alkyle, éther ou aryle étant éventuellement fonctionnalisés par, par exemple, des halogènes, des esters carboxyliques, sulphoniques, des esters de sulfate, des esters phosphoniques, des esters de phosphate. Conviennent bien les substituants hydrophiles qui assurent une bonne solubilité du réactif dans des mélanges d'eau avec des solvants organiques.

Le substituant hydrophile assure généralement qu'une solution du réactif dans un mélange dioxanne/eau 1 :1 en volume est homogène à 20 °C lorsque la concentration du réactif est d'au moins 0.5*10⁻³ mol/l. Souvent la concentration est d'au moins 1*10⁻³ mol/l. De préférence la concentration est d'au moins 0.5*10⁻² mol/l. De bons substituants hydrophiles assurent que la solution est homogène à 20 °C lorsque la concentration du réactif est d'au moins 1*10⁻² mol/l.

On préfère mettre en oeuvre un substituant qui contient au moins une liaison éther. Des exemples de substituants contenant au moins une liaison éther sont, par exemple, les éthers d'alkyle ou d'aryle de mono- oligo- ou de polyalkylèneglycols tels que par exemple le mono- oligo ou polyéthylèneglycol ou le mono- oligo- ou polypropylèneglycol. Le substituant 2-méthoxyéthyl est particulièrement préféré.

Des variantes particulièrement préférées du réactif selon l'invention comprenant un substituant hydrophile répondent à la formule générale (I) 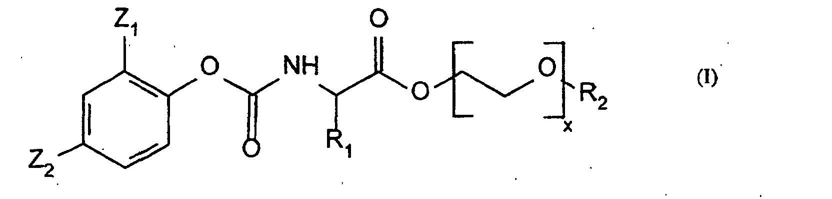 dans laquelle Z₁ et/ou Z₂ = NO2 R₁= Phényl, α ou β-Indolyl, 1-Naphtyl ou 2-Naphtyl, R₂ = Me, Et, C3-C6 alkyl ou cycloalkyl et x représente un nombre intégral de 1 à 5.

Dans une variante, le substituant par lequel le groupement carboxyle de l'aminoacide est substitué, comprend au moins un chromophore. Par chromophore on entend désigner un groupement fonctionnel qui absorbe du rayonnement électromagnetique. Généralement, le maximum d'absorption des chromophores est de 170 à 2500 nm. De préférence le maximum d'absorption des chromophores est de 200 à 1000 nm. Des exemples de chromophores utilisables sont des systèmes aromatiques, éventuellement substitués en position 2 ou 4 par un substituant -I, -M tel que décrit plus haut. Parmi les substituants comprenant au moins un chromophore, les groupements 4-nitrobenzyl, (2-anthraquinoyl)méthyl et (9(9H-fluorénylméthyl)) sont particulièrement préférés.

Des variantes préférées du réactif selon l'invention comprenant au moins un chromophore, répondent à la formule générale (II) 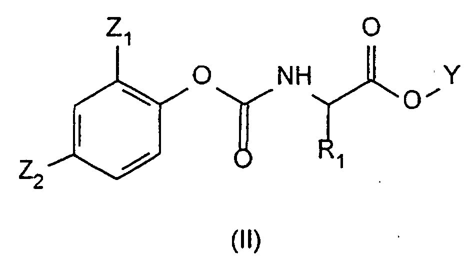 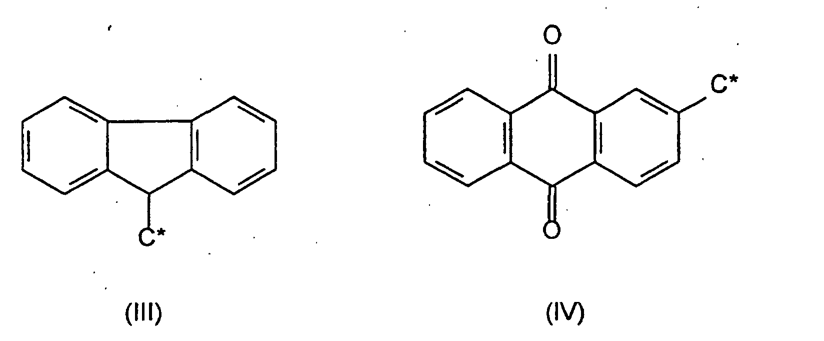 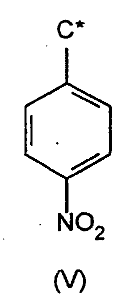 dans laquelle Z₁ et/ou Z₂ = NO2 R₁= Phényl, α ou β-Indolyl, 1-Naphtyl ou 2-Naphtyl, et Y correspond à l'une quelconque des formules (III à V), le carbone par lequel Y est lié à l'oxygène du groupement carboxyle de l'aminoacide énantiopur étant marqué par *.

On peut mettre en oeuvre un substituant hydrophile comprenant au moins un chromophore.

Des variantes particulièrement préférées du réactif selon l'invention comprenant un précurseur actif d'un groupement isothiocyanate et un substituant hydrophile répondent à la formule générale (VI) 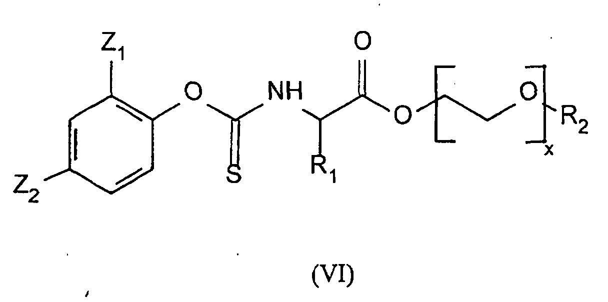 dans laquelle Z₁ et/ou Z₂ = NO2 ou F, R₁= Phényl, α ou β-Indolyl, 1-Naphtyl ou 2-Naphtyl, R₂ = Me, Et, C3-C6 alkyl ou cycloalkyl et x représente un nombre intégral de 1 à 5.

Des variantes préférées du réactif selon l'invention comprenant un précurseur actif d'un groupement isothiocyanate et au moins un chromophore, répondent à la formule générale (VII) 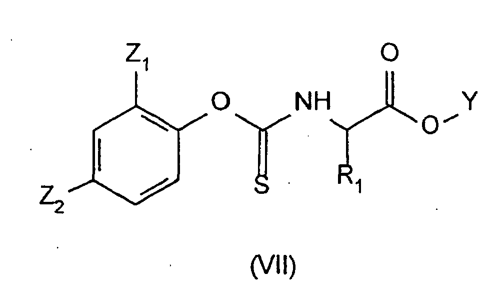 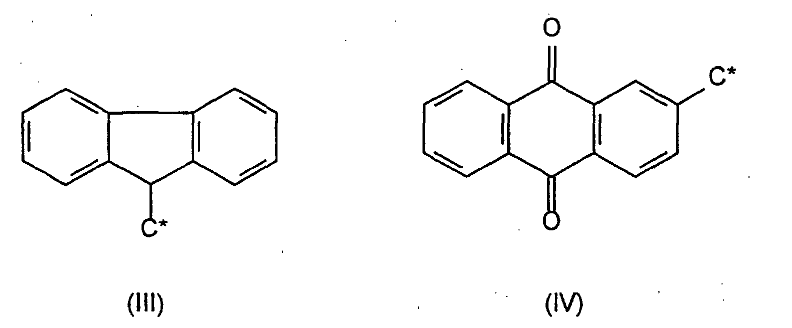 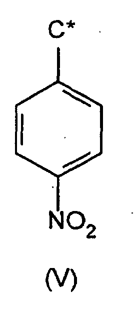 dans laquelle Z₁ et/ou Z₂ = NO2 R₁= Phényl, α ou β-Indolyl, 1-Naphtyl ou 2-Naphtyl, et Y correspond à l'une quelconque des formules (III à V), le carbone par lequel Y est lié à l'oxygène du groupement carboxyle de l'aminoacide énantiopur étant marqué par *.

Lorsque l'aminoacide énantiopur contient plus d'un groupement carboxyle, on préfère protéger tous les groupements carboxyles. De manière particulièrement préférée, tous les groupements carboxyles sont substitués par un substituant tel que décrit plus haut.

Lorsque des groupements fonctionnels libres sont présent dans l'aminoacide énantiopur, on préfère protéger lesdits groupements par des techniques connues en elles.

Le réactif selon l'invention peut être obtenu à partir de l'aminoacide énantiopur respectif. Des méthodes connues sont utilisables pour effectuer l'estérification d'au moins un groupement carboxyle de l'aminoacide. On peut, par exemple, mettre en oeuvre l'aminoacide énantiopur et l'alcool correspondant au substituant à introduire dans un solvant organique tel que par exemple le toluène ou le benzène, en présence d'acide p-toluènesulphonique, de préférence dans des conditions d'estérification azéotropique. Par cette voie de synthèse on obtient des dérivés de tosylate d'ammonium d'ester d'aminoacide, qui conviennent bien pour introduire un groupement activant pour former un précurseur actif d'un groupement isocyanate.

A titre d'exemple de l'introduction d'un groupement activant, on cite la réaction d'un chlorure d'aryloxycarbonyl avec le groupement -NH₂, éventuellement converti en dérivé d'ammonium, d'un aminoacide ou d'un ester d'aminoacide en milieu basique ou neutre dans un solvant organique polaire. A titre de base conviennent notamment des bases d'amine tertiaires tels que par exemple la triéthylamine ou la pyridine. Quand on travaille en milieu neutre, on préfère mettre en oeuvre de l'hydrogènecarbonate de sodium. Ainsi des bons résultats sont obtenus lorsqu'on fait réagir un tosylate d'ammonium d'ester d'aminoacide avec du chlorure de p-nitrophényloxycarbonyl en présence d'hydrogènecarbonate de sodium dans un solvant organique polaire tel que l'acétonitrile.

Dans le procédé selon l'invention on fait réagir le réactif selon l'invention en milieu basique avec un mélange comprenant au moins des énantiomères comprenant au moins un groupement fonctionnel libre.

Le schéma 1 ci-après illustre de façon non limitative la réaction d'un réactif selon l'invention, obtenu à partir de l'ester 2-méthoxyéthyl de la L-phénylalanine et du chlorure de (4-nitrophénoxy)formiate avec un aminoacide. Les produits de cette réaction sont des urées comprenant deux aminoacides dans lesquels au moins une fonction carboxyle est substituée avec un substituant.

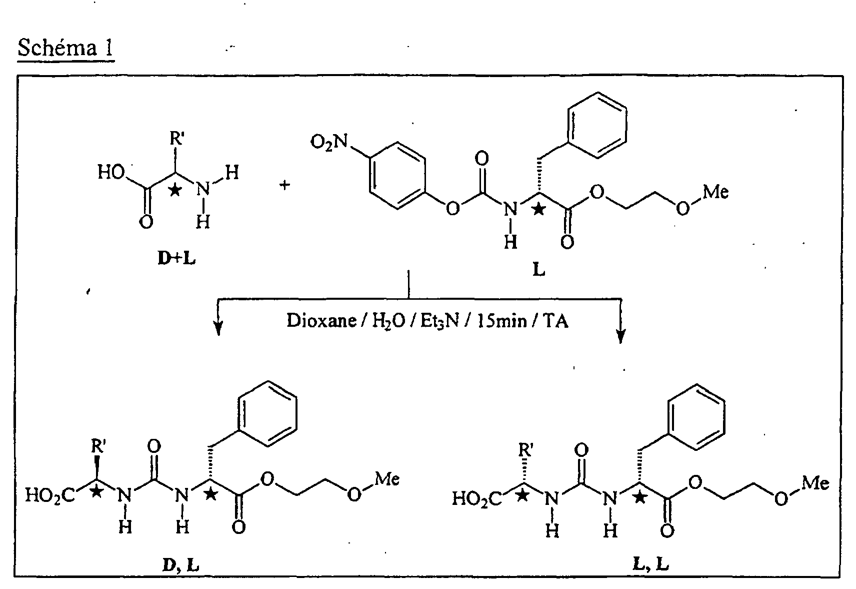

Généralement, le groupement fonctionnel libre est un groupement amino, éventuellement monoalkylé, un groupement hydroxyle ou un groupement thiol. Le groupement fonctionnel libre peut également être constitué d'un anion tel que par exemple un carbanion ou un énolate. Les énantiomères comprenant au moins un groupement fonctionnel libre séparables par le procédé selon l'invention sont généralement des aminoacides, des amines primaires ou secondaires, des peptides, des alcools, des hydroxyacides ou des thiols. Le procédé selon l'invention donne de bons résultats pour séparer les énantiomères d'aminoacides tels que, par exemple, les aminoacides d'origine naturelle ou synthétique mentionnés plus haut.

Le procédé selon l'invention donne de bons résultats également pour séparer un mélange d'énantiomères d'iminoacides. Par iminoacide on entend désigner tout composé comprenant au moins un groupement NHR, dans lequel R représente un radical organique tel que par exemple un radical alkyle ou aryle, et au moins un groupement carboxyle. De tels iminoacides sont par exemple ceux appartenant au groupe constitué de Proline, Acide pipécolique (Acide pipéridine-2-carboxylique), Acide morpholine-3-carboxylique, Acide pipérazine-2-carboxylique, Acide 1-Thia-4-azacyclohexane-3-carboxylique, α-Méthylproline, Cis-4 -hydroxyproline, Baïkaine (Acide 1,2,3,5-tétrahydropyridine-2-carboxylique), Acide Cis-4 -hydroxypipécolique, Acide Trans-5 -hydroxypipécolique, Acide 1,2,3,4-tetrahydronorharmane-1-carboxylique, Acide 1,2,3,4-tétrahydro-6 -hydroxyisoquinoline-3-carboxylique, Acide 1,2,3,4-tétrahydroisoquinoline-3-carboxylique, Acide 1,2,3,4-tétrahydroisoquinoline-1-carboxylique et N-Méthylvaline.

Le procédé selon l'invention est effectué dans un système de solvant, dans lequel le mélange d'énantiomères et le réactif possèdent une solubilité suffisante et le groupement fonctionnel libre possède une nucléophilicité suffisante pour réagir avec un isocyanate. Conviennent, par exemple, à titre de système de solvant des systèmes comprenant au moins un solvant organique polaire et éventuellement de l'eau. Des solvants organiques polaires utilisables sont, par exemple les éthers aliphatiques ou alicycliques tels que le diéthyléther, le tétrahydrofuranne ou le 1,4-dioxanne ainsi que les esters aliphatiques tels que par exemple l'acétate d'éthyle, les amides secondaires aliphatiques tels que par exemple la diméthylformamide et la diméthylacétamide ou par exemple la N-méthylpyrrolidone ou l'acétonitrile.

De bons résultats pour des composés organiques comprenant un groupement fonctionnel libre tel qu'un groupement amino, éventuellement monoalkylé, un groupement hydroxyl arylique ou un groupement thiol sont obtenus dans un système de solvant comprenant un solvant organique polaire et de l'eau. Le dioxanne est préférée à titre de solvant organique polaire. Le rapport pondéral entre le solvant organique polaire et l'eau dans le système de solvant est généralement inférieur ou égal à 99 :1. Le plus souvent, le rapport est inférieur ou égal à 75 :25. Le rapport est généralement supérieur ou égal à 1 :99. Le plus souvent, le rapport est supérieur ou égal à 25 :75.

L'invention concerne aussi une solution du réactif selon l'invention dans un solvant organique polaire tel que par exemple les solvants organiques polaires décrits ci-avant. La concentration du réactif dans la solution est généralement d'au moins 1.10⁻³ mol/l. De préférence, la concentration est d'au moins 1.10⁻² mol/l. La concentration du réactif dans la solution est généralement d'au plus 1.10⁻¹ mol/l. De préférence, la concentration est d'au plus 6.10⁻² mol/l. Dans la solution selon l'invention on préfère utiliser un solvant organique polaire de pureté analytique. Si souhaitable, la solution selon l'invention peut contenir des additifs tels que, par exemple, des stabilisants.

L'invention concerne également l'utilisation de la solution selon l'invention dans un appareil automatique de dérivatisation et de séparation d'énantiomères de composés organiques comprenant au moins un groupement fonctionnel libre. La quantité du réactif à mettre en oeuvre dépend du nombre de groupements fonctionnels libres dans le composé organique. On met en oeuvre au moins 1 équivalent molaire de réactif par groupement fonctionnel libre. Généralement, on met en oeuvre au plus 10 équivalents molaires de réactif par groupement fonctionnel libre. Le plus souvent, on met en oeuvre au plus 5 équivalents molaires de réactif par groupement fonctionnel libre. De préférence, on met en oeuvre au plus 3 équivalents molaires de réactif par groupement fonctionnel libre. De manière particulièrement préférée, on met en oeuvre de 1,1 à 2,5 équivalents molaires de réactif par groupement fonctionnel libre.

Le caractère basique du milieu réactionnel est généré par des méthodes connues. On travaille, de préférence, en présence d'au moins une base. A titre de base conviennent notamment des bases d'amine tertiaires tels que par exemple la triéthylamine ou la diisopropyléthylamine, qui comprennent une fonctionnalité basique respectivement ou la N,N,N,N-tétraméthyléthylènediamine qui comprend 2 fonctionnalités basiques.

La quantité de base à mettre en oeuvre dépend de la quantité du réactif et du nombre de fonctionnalités basiques dans la base. Le rapport molaire entre le réactif et les fonctionnalités basiques est généralement d'au moins 1. Le rapport est généralement d'au plus 2. Un rapport de 1 donne de bons résultats.

Dans le procédé selon l'invention, la durée pendant laquelle on fait réagir le réactif avec le mélange comprenant les énantiomères est généralement inférieure ou égale à 30 min. Le plus souvent, la durée est inférieure ou égale à 20 min. De préférence la durée est inférieure ou égale à 15 min. De bons résultats sont obtenus avec une durée supérieure ou égale à 15 secondes. En pratique, on applique le plus souvent une durée supérieure ou égale à 1 min. Une durée de 5 à 15 min convient bien.

La température à laquelle on fait réagir le réactif avec le mélange comprenant au moins les énantiomères d'un composé organique est généralement inférieure ou égale à 35 °C. Le plus souvent, la température est inférieure ou égale à 30 °C. La température est généralement supérieure ou égale à -20 °C. Le plus souvent, la température est supérieure ou égale à 0 °C. De façon particulièrement préférée, la température est la température ambiante, c'est-à-dire généralement de 15 à 30 °C, de préférence 20 à 25 °C.

Dans le procédé selon l'invention, on soumet le mélange de diastéréomères obtenu à une opération de séparation. Les opérations de séparation utilisables pour la séparation d'un mélange de diastéréomères sont décrites par exemple dans E. Eliel, Stereochemistry of Organic compounds, 1994, p. 344-381. A titre d'exemple citons les opérations de distillation, de cristallisation et de chromatographie gazeuse ou liquide. Parmi ces opérations, les opérations de chromatographie liquide telles que par exemple la chromatograpie HPLC sont préférées. De façon particulièrement préférée, l'opération de séparation est une chromatographie HPLC-RP (phase inverse). Des informations concernant la chromatographie HPLC sont contenues, par exemple, dans RÖMPP CHEMIE-LEXIKON, 9. Ed., p. 1860-1861. On peut également mettre en oeuvre la chromatographie en couche mince.

Des éluants utilisables dans une opération de chromatographie sont connus. Dans le cas où le procédé selon l'invention comprend à titre d'opération de séparation une chromatographie HPLC-RP, de bons résultats ont été obtenu avec un éluant comprenant de l'acétonitrile ou du méthanol.

Dans une variante du procédé selon l'invention, qui est préférée, on soumet le mélange de diastéréomères obtenu à l'opération de séparation sans épuration préalable. Dans des méthodes de séparation d'énantiomères connues, on isole un mélange brut de diastéréomères qui doit être soumis à une épuration préalablement à la séparation des diastéréomères. Le procédé et le réactif selon l'invention permettent de ne pas isoler le mélange brut de diastéréomères et d'effectuer l'opération de séparation sans épuration préalable.

Le procédé et le réactif selon l'invention peuvent être utilisés pour la séparation préparative ou analytique d'énantiomères. Le procédé et le réactif conviennent bien pour la séparation analytique d'énantiomères. Dans une variante, le procédé et le réactif sont utilisés pour déterminer l'excès énantiomérique d'un aminoacide ou d'une amine primaire ou secondaire. Dans une autre variante, le procédé ou le réactif sont utilisés pour déterminer l'excès énantiomérique d'un peptide.

L'invention concerne également un procédé pour l'obtention d'un composé énantiopur comprenant au moins un groupement fonctionnel libre dans lequel
(a) on soumet un mélange comprenant les énantiomères du composé comprenant au moins un groupement fonctionnel libre au procédé de séparation selon l'invention
(b) on effectue une opération de clivage d'un diastéréomère pur obtenu par séparation du mélange de diastéréomères
(c) on récupère le composé énantiopur.

A titre d'opération de clivage on peut utiliser, par exemple, une opération d'hydrazinolyse dans un solvant tel que, par exemple, un alcool.

Lorsqu'on utilise le procédé ou le réactif selon l'invention pour la séparation analytique d'énantiomères, on met en oeuvre une technique de détection connue en elle pour la détermination de la teneur du mélange en énantiomères. Conviennent bien à titre de technique de détection, les techniques optiques telles que par exemple la spectrométrie UV, la spectrométrie visible ou la fluorimétrie.

Les exemples ci-après entendent illustrer l'invention sans toutefois la limiter.

### Exemple 1 (Synthèse du réactif selon l'invention)

### Etape A

Dans un ballon monocol, on a introduit 1 équivalent d'aminoacide énantiopur, 1,5 équivalent d'acide paratoluène sulfonique monohydrate, 50 équivalents de toluène PA et 5 équivalents de Méthoxyéthanol. Le mélange a été chauffé au reflux et l'eau a été éliminée au moyen d'un Dean-Stark. Après 4 heures de reflux, le mélange réactionnel a été refroidi à température ambiante et dilué par de l'éther éthylique. Le mélange réactionnel dilué a été placé au frigo pendant 16 heures. Le solide blanc obtenu a été filtré, lavé à l'éther et séché sous vide.

### Etape B

Dans un ballon monocol, on a pesé du NaHCO₃ (2.6 équivalents) et on a introduit sous courant d'azote l'acétonitrile (5.10⁻³ mole dans 36 ml). Le mélange a été refroidi à 0 °C et on a introduit successivement du (4-nitrophénoxy)chloroformate (1 équivalent) suivi du sel d'ammonium de l'aminoacide énantiopur obtenu selon exemple 1, étape A (1 équivalent). Le mélange a été agité vigoureusement 1 heure à 0 °C et a été ensuite remonté à température ambiante pendant 4 heures. Ce temps écoulé, le mélange a été transféré dans une ampoule à décanter, a été dilué par une solution 1 molaire d'HCl et extrait trois fois à l'éther. Les phases organiques combinées ont été séchées sur MgSO₄, filtrées et concentrées sous pression réduite. Le produit brut a été soumis à un traitement d'épuration approprié.

### Exemple 2

On a effectué les étapes A et B en mettant en oeuvre la L-phénylalanine à titre d'aminoacide énantiopur. Le produit brut a été recristallisé dans l'isopropanol jusqu'à l'obtention de la pureté désirée. Le rendement de l'étape B a été 64 %. On a obtenu un solide cristallin qui était stable au stockage à température ambiante.

Le réactif ainsi obtenu a présenté les données analytiques suivantes :

| | |
|---|---|
| RMN (H¹) | (référence Dioxanne à 3,71 ppm; produit dissous dans le dioxanne-d6) |
| 8,40 (2H, d) | 2H du (4-nitrophenyloxy)carbonyl |
| 7,44 (7H, m) | 2H du (4-nitrophenyloxy)carbonyl et 5H du phényl |
| 7,31 (1H, d) | NH du carbamate |
| 4,83 (1H, m) | CH dé la Phénylalanine |
| 4,41 (2H, m) | CH₂OC=O |
| 3,70 (2H, m) | CH₂OMe |
| 3,47 (3H, s) | CH₃O |
| 3,27 (2H, AB) | 2H benzyliques |

La Résonance magnétique nucléaire (RMN) a été effectuée, dans tous les cas pour lesquels un spectre RMN est indiqué, avec un spéctromètre Appareil Brücker AMX 500 MHz. Les déplacements chimiques sont indiqués en ppm par rapport à la résonance du TMS (tétraméthylsilane). Pour les allures des résonances les abréviations suivantes sont utilisées : m=multiplet, s=singulet, d=doublet, t=triplet, q=quadruplet. Le nombre nH indique le nombre de protons correspondant au signal.
Point de fusion : 71-72 °C
Rotation optique : [α] : + 42,608 (c=0,86 gr/100 ml dans le dioxanne; T° = 26 °C, λ = 589 nm)

La chromatographie en couche mince (CCM) a été effectuée en utilisant des plaques de silicagel MERCK® 60F-254. Rapport frontal = 0,45 (Eluant Diéthyléther/Ether de pétrole : mélange 75/25 en volume).

### Exemples 3-48 (Dérivatisation et séparation de mélanges d'énantiomères)

Dans un vial de 10 ml, on a pesé précisément le mélange d'énantiomères d'un composé organique comprenant au moins un groupement fonctionnel libre selon les exemples 3-48 (voir tableau) (1 équivalent) que l'on a dissout dans de l'eau distillée (concentration 5.10⁻³ mol/l) en présence de triéthylamine. Le rapport molaire de la triéthylamine par rapport au réactif a été 1. Dans un second vial de 10 ml, on a introduit le réactif obtenu selon l'exemple 1 dans lequel l'aminoacide énantiopur était la L-phénylalanine. Le réactif a été dissout dans du dioxanne de pureté analytique (concentration 3.10⁻² mol/l). On a transféré via seringue, et sous agitation vigoureuse, la solution aqueuse de l'acide aminé dans la solution du réactif de dérivatisation. Après 15 minutes d'agitation à température ambiante, on a prélévé 500 µl du mélange réactionnel que l'on a dilué par 500 µl de dioxanne. Cette solution a été analysée par HPLC en phase inverse par injection de 10 µl de solution sur une colonne VYDAC®.

Les suivantes conditions standards ont été utilisées pour les exemples de séparation de mélanges d'énantiomères par HPLC.

On a utilisé une colonne VYDAC® Reverse Phase C18 CAT 201 TP54.

On a employé pour l'élution un mélange Acétonitrile / H₂O avec un gradient en acétonitrile de 1.58 % / min en présence de 0.1 % en volume d'acide trifluoroacétique.

La détection a été effectuée par spectrométrie UV à 205 et 220 nm.

Le tableau 1 ci-après rassemble les mélanges d'énantiomères qui ont été mis en oeuvre avec le réactif obtenu selon l'exemple 1 et les résultats de séparation obtenus.

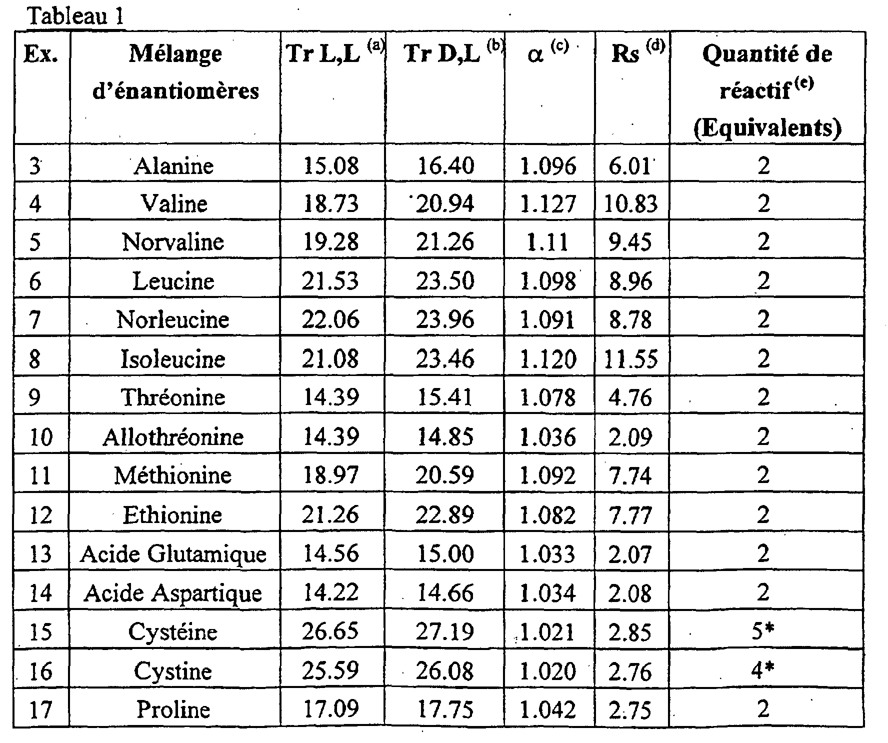

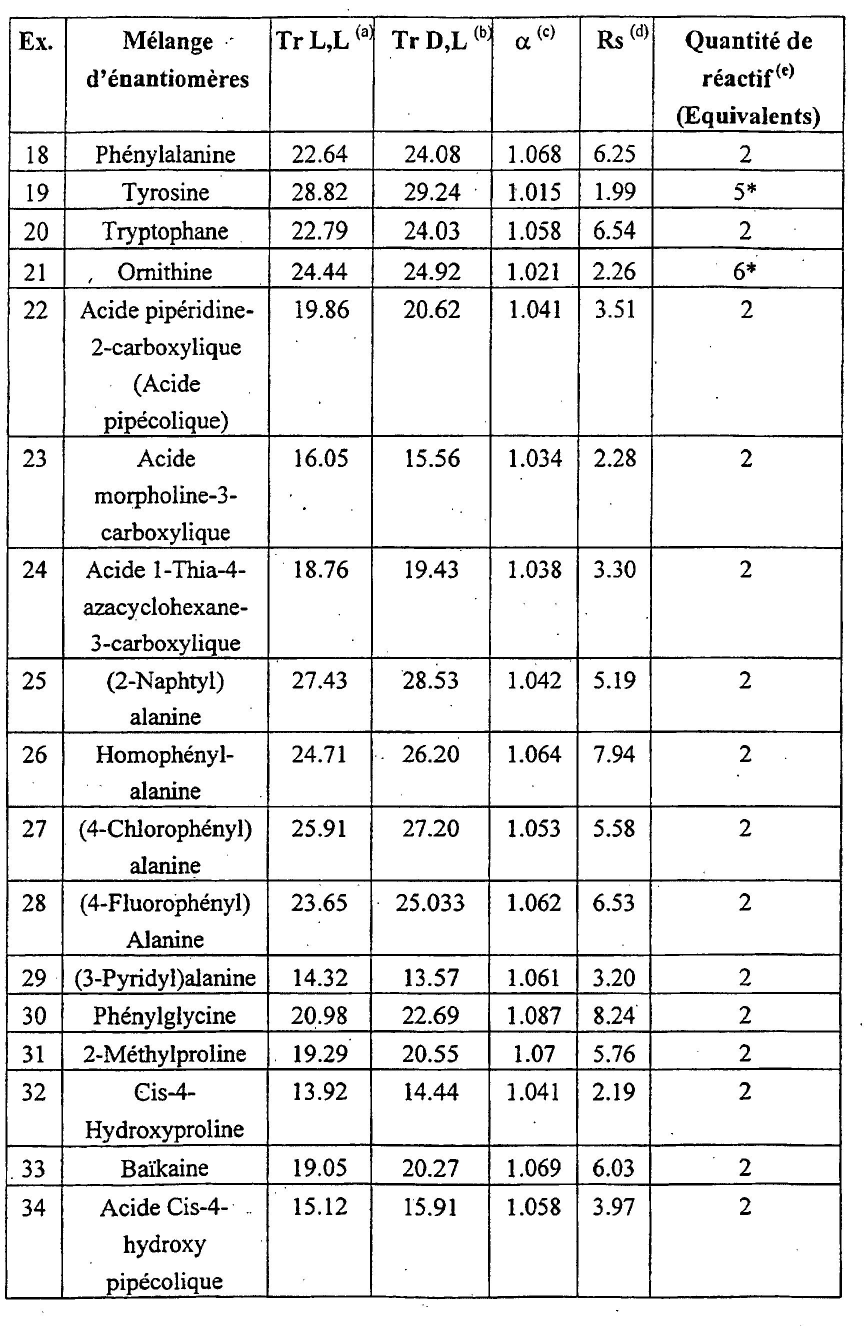

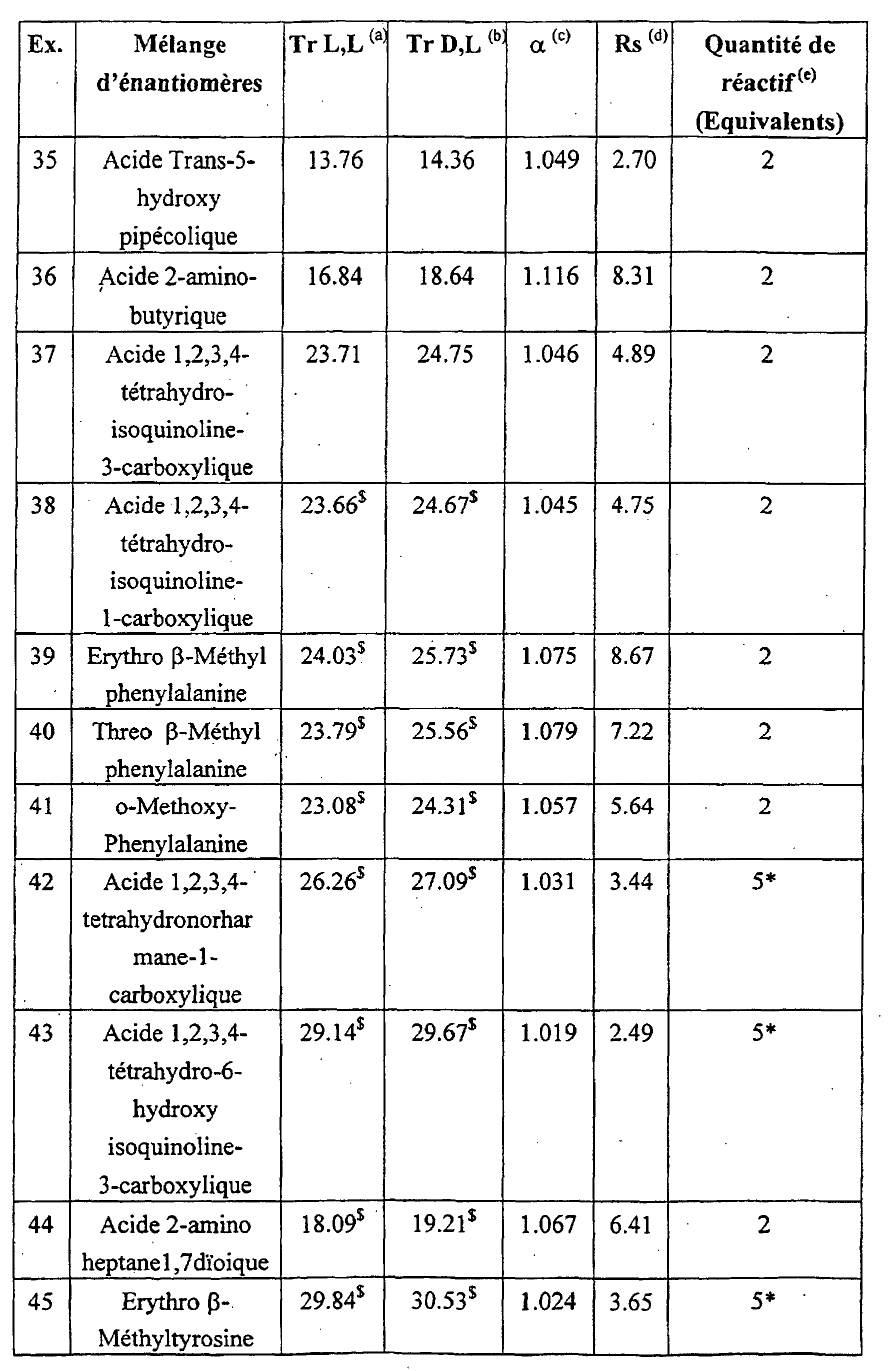

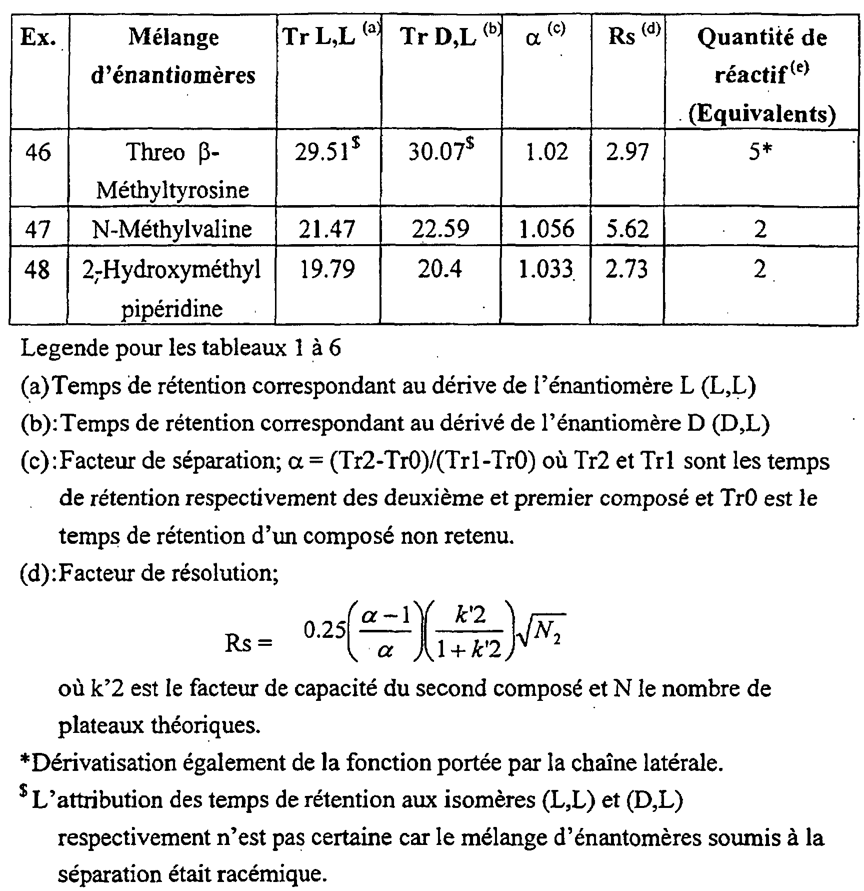

### Exemple 49

On a effectué les étapes A et B en mettant en oeuvre la L-(2-naphtyl)alanine à titre d'aminoacide énantiopur. Le produit brut a été recristallisé dans l'isopropanol jusqu'à l'obtention de la pureté désirée. Le rendement de l'étape B a été 77 %. On a obtenu un solide cristallin qui était stable au stockage à température ambiante.

On a obtenu le spectre RMN suivant du réactif :

| | |
|---|---|
| RMN (H1) | (référence Dioxanne à 3,71 ppm; produit dissous dans le dioxanne-d6) |
| 8,20 (2H, d) | 2H du (4-nitrophenyloxy)carbonyl |
| 7,79 (4H, m) | 4H du naphtyl |
| 7,45 (3H, m) | 3H du naphtyl |
| 7,21 (3H, m) | 2H du (4-nitrophenyloxy)carbonyl et 1H du naphtyl |
| 4,78 (1H, m) | CH du Naphtylalanine |
| 4,27 (2H, m) | CH20C=O |
| 3,52 (2H, m) | CH2OMe |
| 3,28 (3H, s) | CH3O |
| 3,29 (2H, AB) | 2H benzyliques |
| Point de fusion | 76-77 °C |
| Rotation optique | [α] : 83,5 (c = 1,025 gr/100 ml dans le dioxanne; T° = 26 °C, λ = 589 nm) |

La chromatographie en couche mince (CCM) a été effectuée en utilisant des plaques de silicagel MERCK® 60F-254. Ancien Rapport frontal = 0,4 (Eluant Diéthyléther/Ether de pétrole : mélange 75/25 en volume).

On a effectué la réaction de l'ester 2-méthoxyéthyl de la L-N-(4-nitrophénoxy)carbonyl-(2-naphtyl)alanine avec un mélange d'énantiomères d'arginine et l'opération de séparation dans les conditions des exemples 3-48.

On a mis en oeuvre 2 équivalents de réactif et 2 équivalents de triéthylamine par équivalent d'arginine.

Le tableau 2 ci-après montre le résultat de séparation obtenu.

**Tableau 2**

| **Exemple** | **Mélange d'énantiomères** | **Tr L,L** ^{**(a)**} | **Tr D,L** ^{**(b)**} | **α** ^{**(c)**} | **Rs** ^{**(d)**} | **Quantité de réactif**^{**(e)**} **(Equivalents)** |
|---|---|---|---|---|---|---|
| 49 | Arginine | 20.168 | 19.625 | 1.030 | 1.73 | 2 |

### Exemple 50

On a effectué la réaction de l'ester 2-méthoxyéthyl de la L-N-(4-nitrophénoxy)carbonyl-β-tryptophane avec un mélange d'énantiomères de valine et l'opération de séparation dans les conditions des exemples 3-48. On a mis en oeuvre 2 équivalents de réactif et 2 équivalents de triéthylamine par équivalent de valine.

Le tableau 3 ci-après montre le résultat de séparation obtenu.

**Tableau 3**

| **Exemple** | **Mélange d'énantiomères** | **Tr L,L** ^{**(a)**} | **Tr D,L** ^{**(b)**} | **a** ^{**(c)**} | **Rs** ^{**(d)**} | **Quantité de réactif**^{**(e)**} **(Equivalents)** |
|---|---|---|---|---|---|---|
| 50 | Valine | 19.19 | 20.42 | 1.069 | 5.80 | 2 |

### Exemple 51 : Préparation de l'ester 4-nitrobenzyle de la N-((4-nitrophénoxy)carbonyl de phenylalanine)

### Etape A :

Dans un ballon monocol, on a introduit 4 gr (1 éq.) de Z-(L)-Phénylalanine, 2,89 gr (1 éq.) de bromure de 4-nitrobenzyle et 26 ml (25 éq.) de diméthylformamide. On a additionné alors sous azote 1.55 gr (2 éq.) de fluorure de potassium sec. Le mélange a été agité à 60 °C pendant 16 heures. Le mélange réactionnel a été refroidi à température ambiante et dilué par 100 ml d'acétate d'éthyle. La phase organique a été lavée par deux fois 100 ml d'une solution de NaHCO₃ à 5 % ainsi que par deux fois 100 ml d'eau. La phase organique a été séchée sur MgSO₄, filtrée et concentrée sous pression réduite. Le solide obtenu a été séché à l'étuve une nuit. Rendement : 6,3 gr (85 %)

### Etape B :

Le solide obtenu dans l'étape 1 a été dilué dans 25 ml d'acide acétique glacial. On a ajouté prudemment 6,9 ml (3 éq.) d'une solution à 33 % en poids d'HBr dans l'acide acétique. Le mélange est agité une heure et demi à température ambiante. Le mélange a été dilué par 200 ml d'éther éthylique et le précipité blanc formé a été filtré et lavé par trois fois 200 ml d'éther éthylique. Le solide a été séché à l'étuve pendant une nuit.
Rendement : 5,53 gr (99 % )

### Etape C :

Dans un ballon monocol, on a pesé 0,82 gr de NaHCO₃ (2,6 éq.) et on a introduit sous courant d'azote 27 ml d'acétonitrile (135 éq.). Le mélange a été refroidi à 0 °C et on a introduit successivement 0,81 gr (1 éq.) de chlorure de (4-nitrophénoxy)carbonyl suivi de 1,5 gr (1 éq.) du sel de brome du dérivé de la phénylalanine-4-nitrobenzyle. Le mélange a été agité vigoureusement 1 heure à 0 °C et a été ensuite remonté à température ambiante pendant 11 heures. Ce temps écoulé, le mélange a été transféré dans une ampoule à décanter, a été dilué par 60 ml d'une solution 1M d'acide chlorhydrique et a été extrait par trois fois 60 ml d'acétate d'éthyle. Les phases organiques combinées ont été séchées sur MgSO₄, filtrées et concentrées sous pression réduite pour donner un solide beige. Celui-ci a été trituré dans 50 ml d'éther et filtré.

| | |
|---|---|
| Rendement | 1,44 gr (82 %) |
| RMN (H1) | (référence Méthanol à 3,32 ppm; produit dissous dans le Méthanol-d4) |
| 8,32 (2H, d) | 2H du 4-nitrophenoxycarbonyl |
| 8,28 (2H, d) | 2H du 4-nitrobenzyle |
| 7,63 (2H, d) | 2H du 4-nitrophenoxycarbonyl |
| 7,34-7,41 (7H, m) | 2H du 4-nitrobenzyle et 5H du phényl |
| 5,37 (2H, s) | CH2 benzylique du 4-nitrobenzyle |
| 4,65 (1H, m) | CH de la Phénylalanine |
| 3,23 (2H, AB) | CH2 benzyliques de la phényalanine |
| Point de fusion | 118,3 °C |
| Rotation optique | [α] : + 20,83 (c = 0.96 gr/100 ml dans le dioxanne; T° = 24 °C, λ = 589 nm) |

La chromatographie en couche mince (CCM) a été effectuée en utilisant des plaques de silicagel MERCK® 60F-254. Rapport frontal = 0,4 (Eluant Diéthyléther/Ether de pétrole : mélange 75/25 en volume).

### Exemple 52

Le tableau 4 présente le résultat de séparation d'un mélange d'énantiomères de valine qui a été mis en oeuvre avec le réactif obtenu selon l'exemple 51 selon le mode opératoire de l'exemple 3. La détection a été effectuée par spectrométrie UV à 205, 220 et 270 nm.

**Tableau 4**

| **Ex.** | **Mélange d'énantiomères** | **Tr L,L** ^{**(a)**} | **Tr D,L** ^{**(b)**} | **α** ^{**(c)**} | **Rs** ^{**(d)**} **(nm)** | **Equivalents de réactifs** | **Rs (nm)** |
|---|---|---|---|---|---|---|---|
| 52 | Valine | 26.207 | 27.246 | 1.042 | 3.92 (205 nm) | 2 | 4.77 (220 - 270) |

On a observé que ce réactif permet une détection à 220 nm et 270 nm. On a obtenu, à ces longeurs d'ondes, un facteur de séparation plus grand que lorsqu'on a effectué la détection à 205 nm.

### Exemple 53

On a synthétisé suivant le mode opératoire de l'exemple 51, l'ester 2-méthylanthraquinone de la N-((4-nitrophénoxy)carbonyl)phénylalanine. Le tableau 5 présente le résultat de séparation d'un mélange d'énantiomères de valine qui a été mis en oeuvre avec le réactif obtenu selon l'exemple 51 selon le mode opératoire de l'exemple 3.

La détection a été effectuée par spectrométrie UV à 205, 220, 270 et 330 nm.

**Tableau 5**

| **Ex.** | **Mélange d'énantiomères** | **Tr L,L** ^{**(a)**} | **Tr D,L** ^{**(b)**} | **α** ^{**(c)**} | **Rs** ^{**(d)**} **(nm)** | **Equivalents de réactifs** | **Rs (nm)** |
|---|---|---|---|---|---|---|---|
| 53 | Valine | 30.168 | 30.884 | 1.025 | 3.01 (205 nm) | 2 | 3.11 (270 et 330) |

On a observé que ce réactif permet une détection à 270 nm et 330 nm. On a obtenu, à ces longeurs d'ondes, un facteur de séparation légèrement plus grand que lorsqu'on a effectué la détection à 205 nm.

Il apparaît que le réactif selon l'invention peut être obtenu facilement. Le réactif selon l'invention présente une bonne stabilité à température ambiante.

Le procédé selon l'invention permet de séparer une grande variété de composés organiques chiraux comprenant au moins un groupement fonctionnel libre, de manière simple, rapide et dans des conditions uniformes de séparation sans devoir isoler le produit de la réaction préalablement à l'étape de séparation.

### Exemple 54

Dans un ballon monocol, on a pesé du NaHCO3 (2.6 équivalents) et on a introduit sous courant d'azote l'acétonitrile (12.10-3 mole dans 83ml). Le mélange a été refroidi à 0°C et on a introduit successivement du 4-fluorophénoxychlorothionoformate (1 équivalent) suivi du sel d'ammonium d'aminoacide énantiopur obtenu selon exemple 1, étape A (1 équivalent). Le mélange a été agité vigoureusement 1 heure à 0°C et a été ensuite remonté à température ambiante pendant 4 heures. Ce temps écoulé, le mélange a été transféré dans une ampoule à décanter, a été dilué par une solution 1 molaire d'HCl et extrait trois fois à l'éther. Les phases organiques combinées ont été séchées sur MgSO4, filtrées et concentrées sous pression réduite. Le produit brut a été soumis à un traitement d'épuration approprié.
Le réactif ainsi obtenu a été le (S)-N-(4-Fluoro-phenoxythiocarbonyl)-phenylalanine methoxyethyl ester. Il a présenté les données analytiques suivantes :
RMN (H¹): (réf Dioxanne à 3,71ppm; produit dissous dans le dioxanne-d6)
8,88 (2H, d) : 2H du 4-fluorophenoxycarbonyl
7,20-7,49 (7H, m) : 2H du 4-fluorophenoxythiocarbonyl et 5H du phényl
6,99 (1H, d) : NH du carbamate
5,36 (1H, m) : CH de la Phénylalanine
4,42 (2H, m) : CH₂OC=O
3,69 (2H, m) : CH₂OMe
3,48 (3H, s) :CH₃O
3,41 (2H, AB) : 2H benzyliques

### Exemples 55-57

On a effectué la dérivatisation et séparation des mélanges d'énantiomères de composés organiques comprenant un groupement fonctionnel libre des exemples 55-57 (tableau ) dans les conditions des exemples 3-48, en utilisant comme réactif le réactif obtenu dans l'exemple 54. La détection a été effectuée à 245nm uniquement. Le tableau ci-après montre les résultats de séparation obtenus.

**Tableau 6**

| **Exemples** | **Mélange d'énantiomères** | **Tr L,L** ^{(a)} | **Tr D,L** ^{(b)} | **α** ^{**(c)**} | **RS** ^{(d)} | **Quantité de réactif (Equivalents)** |
|---|---|---|---|---|---|---|
| 55 | Valine | 24.79 | 22.11 | 1.129 | 12.89 | 2 |
| 56 | Proline | 19.42 | 21.22 | 1.099 | 7.68 | 2 |
| 57 | Tyrosine | 22.14 | 23.26 | 1.054 | 4.4 | 5 |

Le réactif permet une séparation très efficace d'énantiomères. La détection par spectrométrie UV peut être effectuée à une seule longueur d'ondes et elle est hautement sensible.

## Revendications

1. Procédé pour la séparation d'énantiomères comprenant au moins un groupement fonctionnel libre dans lequel
(a) on fait réagir un mélange comprenant les énantiomères en milieu basique avec un réactif à base d'un aminoacide énantiopur, réactif dans lequel au moins un groupement amino de l'aminoacide porte un groupement activant pour former un précurseur actif d'un groupement isocyanate ou isothiocyanate et dans lequel au moins un groupement carboxyle de l'aminoacide est substitué et
(b) on soumet le mélange de diastéréomères obtenu à une opération de séparation.

2. Procédé selon la revendication 1, dans lequel le groupement carboxyle de l'aminoacide est substitué avec un substituant hydrophile et/ou un substituant comprenant au moins un chromophore.

3. Procédé selon la revendication 2 dans lequel le substituant hydrophile dans le réactif est un groupement 2-méthoxyéthyl.

4. Procédé selon l'une quelconque des revendications 1 à 3, dans lequel le groupement activant dans le réactif est un groupement (4-nitrophényloxy)carbonyl.

5. Procédé selon l'une quelconque des revendications 1 à 4, dans lequel le réactif est à base d'un aminoacide énantiopur sélectionné parmi le groupe constitué de Alanine, Valine, Norvaline, Leucine, Norleucine, Isoleucine, Sérine, Isosérine, Homosérine, Thréonine, Allothréonine, Méthionine, Ethionine, Acide Glutamique, Acide Aspartique, Asparagine, Cystéine, Cystine, Phénylalanine, Tyrosine, Tryptophane, Lysine, Arginine, Histidine, Ornithine, Glutamine, Citrulline, (1-Naphtyl)alanine, (2-Naphtyl)alanine, Homophénylalanine, (4-Chlorophényl)alanine, (4-Fluorophényl)alanine, (3-Pyridyl)alanine, Phénylglycine, Acide Diaminopimélique (Acide 2-6-Diaminoheptane-1,7-dïoique), Acide 2-aminobutyrique, Acide-2-aminotétraline-2-carboxylique, Erythro β-Méthylphenylalanine, Threo β-Méthylphenylalanine, (2-Méthoxyphenyl)alanine, Acide 1-amino-5-hydroxyindane-2-carboxylique, Acide 2-Aminoheptane-1,7-dïoique, (2,6-Diméthyl-4-hydroxyphényl)alanine, Erythro β-Méthyltyrosine et Threo β-Méthyltyrosine.

6. Procédé selon l'une quelconque des revendications 1 à 5 dans lequel on fait réagir le mélange d'énantiomères avec le réactif à température ambiante pendant une durée inférieure ou égale à 15 minutes et on soumet le mélange de diastéréomères obtenu à l'opération de séparation sans épuration préalable.

7. Procédé selon l'une quelconque des revendications 1 à 5, dans lequel l'opération de séparation est une chromatographie HPLC.

8. Réactif à base d'un aminoacide énantiopur dans lequel au moins un groupement amino de l'aminoacide porte un groupement activant pour former un précurseur actif d'un groupement isocyanate ou isothiocyanate et dans lequel au moins un groupement carboxyle de l'aminoacide est substitué, sous réserve que lorsque l'aminoacide est la L-phénylalanine et le groupement activant forme un précurseur actif d'un isocyanate ;
(a) le groupement activant est choisi parmi un groupement hétéroaryloxycarbonyl, 1,3-imidazolyl-N-carbonyl, 1,2,4-triazolyl-N-carbonyl, 4-nitrophényloxycarbonyl ou un groupement aryloxycarbonyl portant un substituant halogène, -SO₂R ,-SO₂OR, NR₂⁺ ou SR₂⁺ à au moins une des positions 2, 4 ou 6 du noyau aromatique ou à des positions analogues au positions 2 ou 4 dans des systèmes aromatiques condensés ; ou
(b) le substituant par lequel le groupement carboxyle de l'arriinoacide est substitué est choisi parmi un groupement méthyl, n-propyl, i-propyl, n-butyl, i-butyl, t-butyl, les éthers et les groupements aryle, éventuellement fonctionnalisés; ou
(c) le substituant par lequel le groupement carboxyle de l'aminoacide est substitué est choisi parmi les groupements alkyles comprenant de 1 à 4 atomes de carbone linéaires ou ramifiés fonctionnalisés de manière à former un substituant hydrophile.

9. Réactif selon la revendication 8, dans lequel au moins un groupement amino de l'aminoacide énantiopur porte un groupement activant pour former un précurseur actif d'un groupement isocyanate.

10. Réactif selon la revendication 8, dans lequel au moins un groupement amino de l'aminoacide énantiopur porte un groupement activant pour former un précurseur actif d'un groupement isothiocyanate.

11. Réactif selon l'une quelconque des revendications 8 à 10, dans lequel l'aminoacide énantiopur est un aminoacide d'origine naturelle.

12. Réactif selon la revendication 11, dans lequel l'aminoacide est choisi parmi Alanine, Valine, Norvaline, Leucine, Norleucine, Isoleucine, Sérine, Isosérine, Homosérine, Thréonine, Allothréonine, Méthionine, Ethionine, Acide Glutamique, Acide Aspartique, Asparagine, Cystéine, Cystine, Phénylalanine, Tyrosine, Tryptophane, Lysine, Arginine, Histidine, Ornithine, Glutamine et Citrulline.

13. Réactif selon l'une quelconque des revendications 8 à 10, dans lequel l'aminoacide énantiopur est un aminoacide d'origine synthétique.

14. Réactif selon la revendication 13, dans lequel l'aminoacide est choisi parmi (1-Naphtyl)alanine, (2-Naphtyl)alanine, Homophénylalanine, (4-Chlorophényl)alanine, (4-Fluorophényl)alanine, (3-Pyridyl)alanine, Phénylglycine, Acide Diaminopimélique (Acide 2-6-Diaminoheptane-1,7-dïoique), Acide 2-aminobutyrique, Acide-2-aminotétraline-2-carboxylique, Erythro β-Méthylphenylalanine, Threo β-Méthylphenylalanine, (2-Méthoxyphenyl)alanine, Acide 1-amino-5-hydroxyindane-2-carboxylique, Acide 2-Aminoheptane-1,7-dïoique, (2,6-Diméthyl-4-hydroxyphényl)alanine, Erythro β-Méthyltyrosine, Threo β-Méthyltyrosine et les énantiomères non-naturels de Alanine, Valine, Norvaline, Leucine, Norleucine, Isoleucine, Sérine, Isosérine, Homosérine, Thréonine, Allothréonine, Méthionine, Ethionine, Acide Glutamique, Acide Aspartique, Asparagine, Cystéine, Cystine, Phénylalanine, Tyrosine, Tryptophane, Lysine, Arginine, Histidine, Ornithine, Glutamine et Citrulline.

15. Réactif selon l'une quelconque des revendications 8 à 14, dans lequel le groupement activant est un groupement aryloxycarbonyl qui porte au moins un substituant nitro sur un noyau aromatique.

16. Réactif selon la revendication 9, répondant à la formule générale (I) 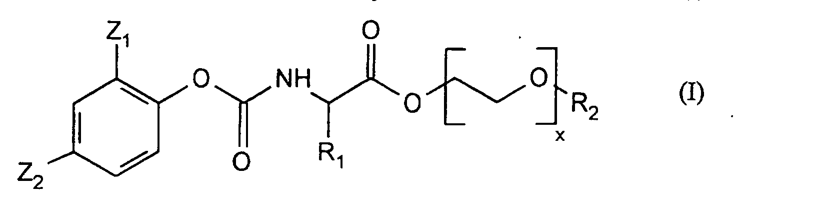 dans laquelle Z₁ et/ou Z₂ = NO2 R₁= Phényl, α ou β-Indolyl, 1-Naphtyl ou 2-Naphtyl, R₂ = Me, Et, C3-C6 alkyl ou cycloalkyl et x représente un nombre intégral de 1 à 5.

17. Réactif selon la revendication 10 comprenant au moins un chromophore, répondant à la formule générale (II) 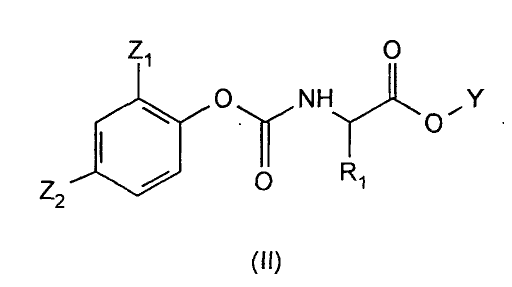 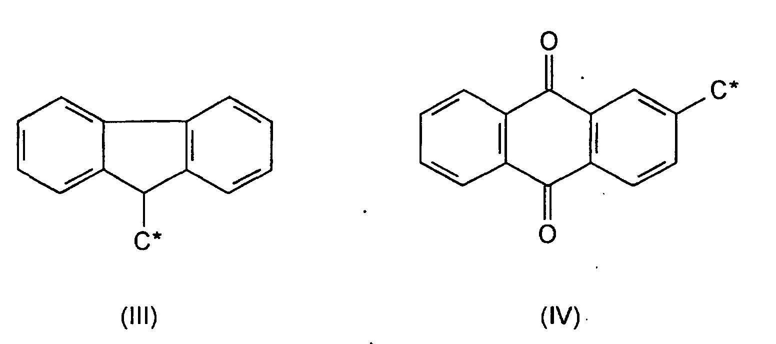 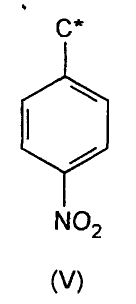 dans laquelle Z₁ et/ou Z₂ = NO2 R₁= Phényl, α ou β-Indolyl, 1-Naphtyl ou 2-Naphtyl, et Y correspond à l'une quelconque des formules III à V, le carbone par lequel Y est lié à l'oxygène du groupement carboxyle de l'aminoacide énantiopur étant marqué par *.

18. Réactif selon la revendication 9 répondant à la formule générale (VI) 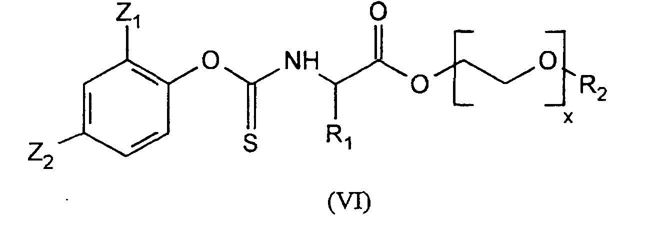 dans laquelle Z₁ et/ou Z₂ = NO2 ou F, R₁= Phényl, α ou β-Indolyl, 1-Naphtyl ou 2-Naphtyl, R₂ = Me, Et, C3-C6 alkyl ou cycloalkyl et x représente un nombre intégral de 1 à 5.

19. Réactif selon la revendication 10 comprenant au moins un chromophore, répondant à la formule générale (VII) 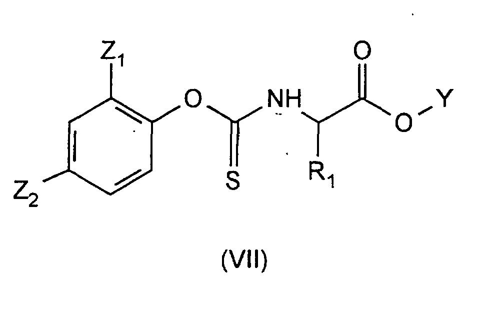 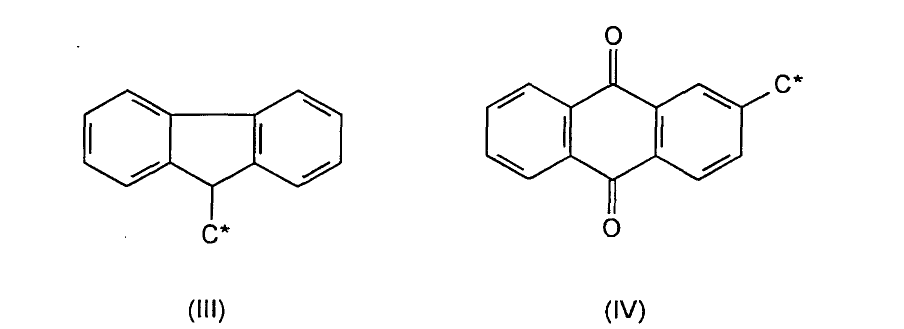 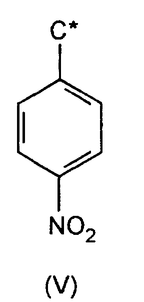 dans laquelle Z₁ et/ou Z₂ = NO2 R₁= Phényl, α ou β-Indolyl, 1-Naphtyl ou 2-Naphtyl, et Y correspond à l'une quelconque des formules III à V, le carbone par lequel Y est lié à l'oxygène du groupement carboxyle de l'aminoacide énantiopur étant marqué par *.

20. Réactif selon l'une quelconque des revendications 8 à 15, dans lequel l'aminoacide énantiopur est la phénylalanine.

21. Réactif selon la revendication 20, répondant à la formule

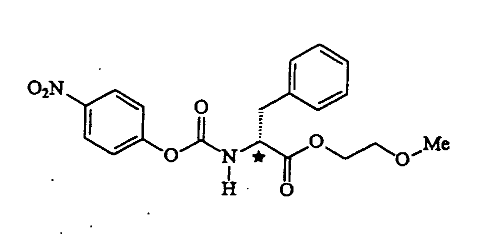

22. Solution du réactif selon l'une quelconque des revendications 8 à 21 dans un solvant organique polaire.

23. Utilisation de la solution selon la revendication 22 dans un appareil automatique de dérivatisation et de séparation d'énantiomères de composés organiques comprenant au moins un groupement fonctionnel libre.

24. Utilisation du procédé, du réactif ou de la solution selon l'une quelconque des revendications 1 à 23 pour la séparation d'énantiomères d'un aminoacide, d'une amine primaire ou secondaire ou d'un peptide.

25. Procédé pour l'obtention d'un composé énantiopur comprenant au moins un groupement fonctionnel libre dans lequel
(a) on soumet un mélange comprenant les énantiomères du composé comprenant au moins un groupement fonctionnel libre au procédé de séparation selon l'une quelconque des revendications 1 à 7
(b) on effectue une opération de clivage d'un diastéréomère pur obtenu par séparation du mélange de diastéréomères
(c) on récupère le composé énantiopur.

## Claims

1. Process for the separation of enantiomers comprising at least one free functional group, in which
(a) a mixture comprising the enantiomers is reacted in basic medium with a reagent based on an enantiopure amino acid, in which reagent at least one amino group of the amino acid carries an activating group, in order to form an active precursor of an isocyanate group, and in which reagent at least one carboxyl group of the amino acid is substituted, and
(b) the mixture of diastereomers obtained is subjected to a separation operation.

2. Process according to Claim 1, in which the carboxyl group of the amino acid is substituted with a hydrophilic substituent and/or a substituent comprising at least one chromophore.

3. Process according to Claim 2, in which the hydrophilic substituent in the reagent is a 2-methoxyethyl group.

4. Process according to any one of Claims 1 to 3, in which the activating group in the reagent is a (4-nitrophenyloxy)carbonyl group.

5. Process according to any one of Claims 1 to 4, in which the reagent is based on an enantiopure amino acid selected from the group consisting of alanine, valine, norvaline, leucine, norleucine, isoleucine, serine, isoserine, homoserine, threonine, allothreonine, methionine, ethionine, glutamic acid, aspartic acid, asparagine, cysteine, cystine, phenylalanine, tyrosine, tryptophan, lysine, arginine, histidine, ornithine, glutamine, citrulline, (1-naphthyl)alanine, (2-naphthyl)alanine, homophenylalanine, (4-chlorophenyl)alanine, (4-fluorophenyl)alanine, (3-pyridyl)alanine, phenylglycine, diaminopimelic acid (2,6-diaminoheptane-1,7-dioic acid), 2-aminobutyric acid, 2-aminotetralin-2-carboxylic acid, erythro-β-methylphenylalanine, threo-β-methylphenylalanine, (2-methoxyphenyl)alanine, 1-amino-5-hydroxyindan-2-carboxylic acid, 2-aminoheptane-1,7-dioic acid, (2,6-dimethyl-4-hydroxyphenyl)alanine, erythro-β-methyltyrosine and threo-β-methyltyrosine.

6. Process according to any one of Claims 1 to 5, in which the mixture of enantiomers is reacted with the reagent at room temperature for a period of time of less than or equal to 15 minutes and the mixture of diastereomers obtained is subjected to the separation operation without prior purification.

7. Process according to any one of Claims 1 to 5, in which the separation operation is HPLC chromatography.

8. Reagent based on an enantiopure amino acid in which at least one amino group of the amino acid carries an activating group in order to form an active precursor of an isocyanate or isothiocyanate group and in which at least one carboxyl group of the amino acid is substituted, provided that when the aminoacid is L-phenylalanine and the activating group forms an active precursor of an isocyanate;
(a) the activating group is chosen from a heteroaryloxycarbonyl group, 1,3-imidazolyl-N-carbonyl group, 1,2,4-triazolyl-N-carbonyl group, 4-nitrophenyloxycarbonyl group or an aryloxycarbonyl group carrying a -SO₂R ,-SO₂OR, NR₂⁺ or SR₂⁺ group at at least one of positions 2, 4 ou 6 of the aromatic nucleus or at positions analogous to positions 2 ou 4 in condensed aromatic systems; or
(b) the substituent by which the carboxyl group of the amino acid is substituted is chosen from methyl group, n-propyl group, i-propyl group, n-butyl group, i-butyl group, t-butyl group, ethers and aryl groups, optionally functionnalised; or
(c) the substituent by which the carboxyl group of the amino acid is substituted is chosen from linear or branched alkyl groups comprising from 1 to 4 carbon atoms which are functionalised to form a hydrophilic substituent.

9. Reagent according to Claim 8, in which at least one amino group of the enantiopure amino acid carries an activating group in order to form an active precursor of an isocyanate group.

10. Reagent according to Claim 8, in which at least one amino group of the enantiopure amino acid carries an activating group in order to form an active precursor of an isothiocyanate group.

11. Reagent according to anyone of Claims 8 to 10, in which the enantiopure amino acid is an amino acid of natural origin.

12. Reagent according to Claim 11, in which the amino acid is chosen from Alanine, Valine, Norvaline, Leucine, Norleucine, Isoleucine, Serine, Isoserine, Homoserine, Threonine, Allothreonine, Methionine, Ethionine, Glutamic Acid, Aspartic Acid, Asparagine, Cysteine, Cystine, Phenylalanine, Tyrosine, Tryptophane, Lysine, Arginine, Histidine, Ornithine, Glutamine and Citrulline.

13. Reagent according to anyone of Claims 8 to 10, in which the enantiopure amino acid is an amino acid of synthetic origin.

14. Reagent according to Claim 13, in which the amino acid is chosen from (1-Naphtyl)alanine, (2-Naphtyl)alanine, Homophenylalanine, (4-Chlorophenyl)alanine, (4-Fluorophenyl)alanine, (3-Pyridyl)alanine, Phenylglycine, Diaminopimelc acid (2-6-Diaminoheptane-1,7-dioic acid), 2-aminobutyric acid, 2-aminotetraline-2-carboxylic acid, Erythro □-Methylphenylalanine, Threo□-Methylphenylalanine, (2-Methoxyphenyl)alanine, 1-amino-5-hydroxyindane-2-carboxylic acid, 2-Aminoheptane-1,7-dioic acid, (2,6-Dimethyl-4-hydroxyphenyl)alanine, Erythro □-Methyltyrosine, Threo□-Methyltyrosine and the non-natural enantiomers of Alanine, Valine, Norvaline, Leucine, Norleucine, Isoleucine, Serine, Isoserine, Homoserine, Threonine, Allothreonine, Methionine, Ethionine, Glutamic Acid, Aspartic Acid, Asparagine, Cysteine, Cystine, Phenylalanine, Tyrosine, Tryptophane, Lysine, Arginine, Histidine, Ornithine, Glutamine and Citrulline.

15. Reagent according to any one of claims 8 to 14, wherein the activating group is an aryloxycarbonyl group which carries at least one nitro substituent on an aromatic ring.

16. Reagent according to Claim 9 corresponding to the general formula (I) 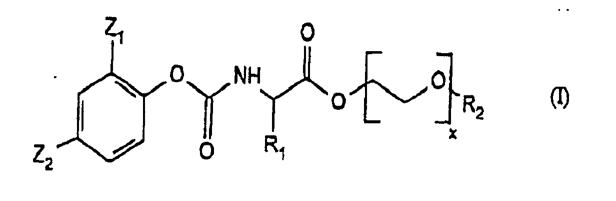 in which Z₁ and/or Z₂ = NO₂, R₁ = phenyl, α- or β-indolyl, 1-naphthyl or 2-naphthyl, R₂ = Me, Et, C₃-C₆ alkyl or C₃-C₆ cycloalkyl, and x represents an integer from 1 to 5.

17. Reagent according to Claim 9, comprising at least one chromophore, corresponding to the general formula (II) 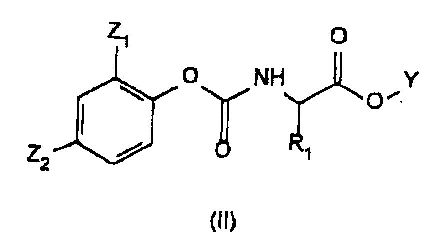 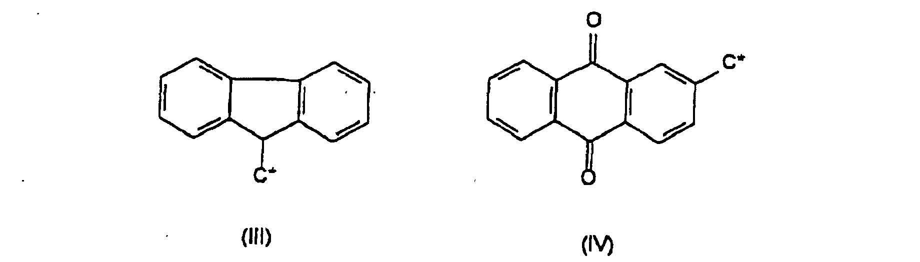 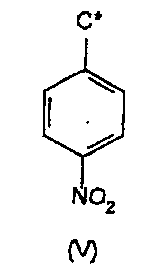 in which Z₁ and/or Z₂ = NO₂, R₁ = phenyl, α- or β-indolyl, 1-naphthyl or 2-naphthyl and Y corresponds to any one of the formulae (III to V), the carbon by which Y is bonded to the oxygen of the carboxyl group of the enantiopure amino acid being marked by *.

18. Reagent according to Claim 10 corresponding to the general formula (VI) 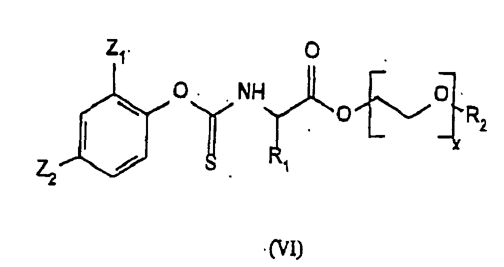 in which Z₁ and/or Z₂ = NO₂ or F, R₁ = phenyl, α- or β-indolyl, 1-naphthyl or 2-naphthyl, R₂ = Me, Et, C₃-C₆ alkyl or C₃-C₆ cycloalkyl and x represents an integer from 1 to 5.

19. Reagent according to Claim 10 comprising at least one chromophore, corresponding to the general formula (VII) 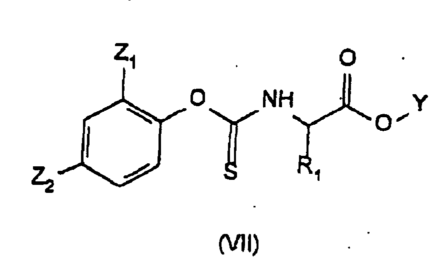 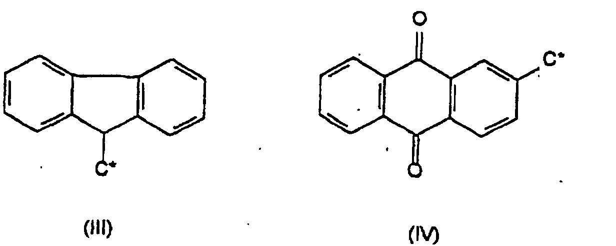 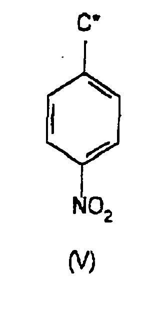 in which Z₁ and/or Z₂ = NO₂, R₁ = phenyl, α- or β-indolyl, 1-naphthyl or 2-naphthyl and Y corresponds to any one of the formulae (III to V), the carbon by which Y is bonded to the oxygen of the carboxyl group of the enantiopure amino acid being marked by *.

20. Reagent according to anyone of claims 8 to 15, wherein the enantiopure aminoacid is phenylalanine.

21. Reagent according to claim 20, corresponding to the formula

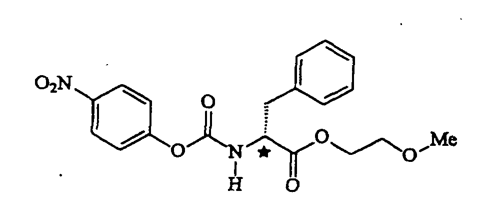

22. Solution of the reagent according to any one of Claims 8 to 21 in a polar organic solvent.

23. Use of the solution according to Claim 22 in an automatic device for the derivatization and separation of enantiomers of organic compounds comprising at least one free functional group.

24. Use of the process, of the reagent or of the solution according to any one of Claims 1 to 23 for the separation of enantiomers of an amino acid, of a primary or secondary amine or of a peptide.

25. Process for the production of an enantiopure compound comprising at least one free functional group in which:
(a) a mixture comprising the enantiomers of the compound comprising at least one free functional group is subjected to the separation process according to anyone of claims 1 to 7
(b) a cleavage operation is carried out on a pure diastereomer obtained by separation of the mixture of diastereomers
(c) the enantiopure compound is recovered.

## Patentansprüche

1. Verfahren zur Auftrennung von Enantiomeren, die wenigstens ein freie funktionelle Gruppe umfassen, worin
(a) ein die Enantiomeren umfassendes Gemisch in basischem Milieu mit einem Reaktionsmittel auf der Basis einer enantiomerenreinen Aminosäure reagiert wird, in welchem Reaktionsmittel wenigstens ein Aminogruppe der Aminosäure ein aktivierende Gruppe trägt, um einen aktiven Vorläufer einer Isocyanat- oder Isothiocyanatgruppe auszubilden und worin wenigstens ein Carboxylgruppe der Aminosäure substituiert ist, und
(b) das erhaltene Diastereomerengemisch einer Trennoperation unterworfen wird.

2. Verfahren nach Anspruch 1, worin die Carboxylgruppe der Aminosäure durch einen hydrophilen Substituenten und/oder einen Substituenten, der wenigstens einen Chromophor umfaßt, substituiert ist.

3. Verfahren nach Anspruch 2, worin der hydrophile Substituent in dem Reaktionsmittel ein 2-Methoxyethylgruppe ist.

4. Verfahren nach einem der Ansprüche 1 bis 3, worin die aktivierende Gruppe in dem Reaktionsmittel ein (4-Nitrophenyloxy)carbonylgruppe ist.

5. Verfahren nach einem der Ansprüche 1 bis 4, worin das Reaktionsmittel auf einer enantiomerenreinen Aminosäure basiert, ausgewählt aus der aus Alanin, Valin, Norvalin, Leucin, Norleucin, Isoleucin, Serin, Isoserin, Homoserin, Threonin, Allothreonin, Methionin, Ethionin, Glutaminsäure, Asparaginsäure, Asparagin, Cystein, Cystin, Phenylalanin, Tyrosin, Tryptophan, Lysin, Arginin, Histidin, Ornithin, Glutamin, Citrullin, (1-Naphtyl)alanin, (2-Naphthyl)alanin, Homophenylalanin, (4-Chlorphenyl)alanin, (4-Fluorphenyl)alanin, (3-Pyridyl)alanin, Phenylglycin, Diaminopimelinsäure (2-6-Diaminoheptan-1,7-dicarbonsäure), 2-Aminobuttersäure, 2-Aminotetralin-2-carbonsäure, erythro-β-Methylphenylalanin, threo-β-Methylphenylalanin, (2-Methoxyphenyl)alanin, 1-Amino-5-hydroxyindan-2-carbonsäure, 2-Aminoheptan-1,7-dicarbonsäure, (2,6-Dimethyl-4-hydroxyphenyl)alanin, erythro-β-Methyltyrosin und threo-β-Methyltyrosin bestehenden Gruppe.

6. Verfahren nach einem der Ansprüche 1 bis 5, worin das Isomerengemisch bei Umgebungstemperatur während einer Dauer von weniger als oder gleich 15 Minuten mit dem Reaktionsmittel reagieren gelassen wird und das erhaltene Diastereomerengemisch ohne vorherige Reinigung der Trennoperation unterworfen wird.

7. Verfahren nach einem der Ansprüche 1 bis 5, worin die Trennoperation ein HPLC-Chromatographie ist.

8. Reaktionsmittel auf der Basis einer enantiomerenreinen Aminosäure, worin wenigstens ein Aminogruppe der Aminosäure ein aktivierende Gruppe zur Ausbildung eines aktiven Vorläufers einer Isocyanat- oder Isothiocyanatgruppe trägt und worin wenigstens ein Carboxylgruppe der Aminosäure substituiert ist, mit der Maßgabe, daß dann, wenn die Aminosäure L-Phenylalanin ist und die aktivierende Gruppe einen aktiven Vorläufer eines Isocyanats ausbildet,
(a) die aktivierende Gruppe unter einer Heteroaryloxycarbonylgruppe, 1,3-Imidazolyl-N-carbonylgruppe, 1,2,4-Triazolyl-N-carbonylgruppe, 4-Nitrophenyloxycarbonylgruppe oder ein Aryloxycarbonylgruppe ausgewählt ist, die einen Halogensubstituenten, -SO₂R, -SO₂OR, NR₂⁺ oder SR₂⁺ an wenigstens einer der Positionen 2, 4 oder 6 des aromatischen Kerns oder an den Positionen 2 oder 4 analogen Positionen in kondensierten aromatischen Systemen trägt; oder
(b) der Substituent, durch den die Carboxylgruppe der Aminosäure substituiert ist, unter einer Methyl-, n-Propyl-, i-Propyl-, n-Butyl-, 1-Butyl-, t-Butylgruppe, den gegebenenfalls funktionalisierten Ethern der Arylgruppen ausgewählt ist; oder
(c) der Substituent, durch den die Carboxylgruppe der Aminosäure substituiert ist, unter den geraden oder verzweigten Alkylgruppen mit 1 bis 4 Kohlenstoffatomen, die derart funktionalisiert sind, daß ein hydrophiler Substituent ausgebildet wird, ausgewählt ist.

9. Reaktionsmittel nach Anspruch 8, worin wenigstens ein Aminogruppe der enantiomerenreinen Aminosäure ein aktivierende Gruppe trägt, um einen aktiven Vorläufer einer Isocyanatgruppe auszubilden.

10. Reaktionsmittel nach Anspruch 8, worin wenigstens ein Aminogruppe der enantiomerenreinen Aminosäure ein aktivierende Gruppe trägt, um einen aktiven Vorläufer einer Isothiocyanatgruppe auszubilden.

11. Reaktionsmittel nach einem der Ansprüche 8 bis 10, in dem die enantiomerenreine Aminosäure eine Aminosäure natürlichen Ursprungs ist.

12. Reaktionsmittel nach Anspruch 11, in dem die Aminosäure aus Alanin, Valin, Norvalin, Leucin, Norleucin, Isoleucin, Serin, Isoserin, Homoserin, Threonin, Allothreonin, Methionin, Ethionin, Glutaminsäure, Aspartinsäure, Asparagin, Cystein, Cystin, Phenylalanin, Tyrosin, Tryptophane, Lysin, Arginin, Histidin, Ornithin, Glutamin und Citrullin ausgewählt ist.

13. Reaktionsmittel nach einem der Ansprüche 8 bis 10, in dem die enantiomerenreine Aminosäure eine Aminosäure synthetischen Ursprungs ist.

14. Reaktionsmittel nach Anspruch 11, in dem die Aminosäure aus (1-Naphtyl)alanin, (2-Naphthyl)alanin, Homophenylalanin, (4-Chlorphenyl)alanin, (4-Fluorphenyl)alanin, (3-Pyridyl)alanin, Phenylglycin, Diaminopimelinsäure (2-6-Diaminoheptan-1,7-dicarbonsäure), 2-Aminobuttersäure, 2-Aminotetralin-2-carbonsäure, erythro-β-Methylphenylalanin, threo-β-Methylphenylalanin, (2-Methoxyphenyl)alanin, 1-Amino-5-hydroxyindan-2-carbonsäure, 2-Aminoheptan-1,7-dicarbonsäure, (2,6-Dimethyl-4-hydroxyphenyl)alanin, erythro-β-Methyltyrosin und threo-β-Methyltyrosin und den nicht natürlichen Enantiomeren von Alanin, Valin, Norvalin, Leucin, Norleucin, Isoleucin, Serin, Isoserin, Homoserin, Threonin, Allothreonin, Methionin, Ethionin, Glutaminsäure, Aspartinsäure, Asparagin, Cystein, Cystin, Phenylalanin, Tyrosin, Tryptophane, Lysin, Arginin, Histidin, Ornithin, Glutamin und Citrullin ausgewählt ist.

15. Reaktionsmittel nach einem der Ansprüche 8 bis 14, in dem die aktivierende Gruppe eine Aryloxycarbonylgruppe ist die wenigstens einen Nitrosubstituenten an einem aromatischen Ring trägt.

16. Reaktionsmittel nach Anspruch 9, entsprechend der allgemeinen Formel (I) 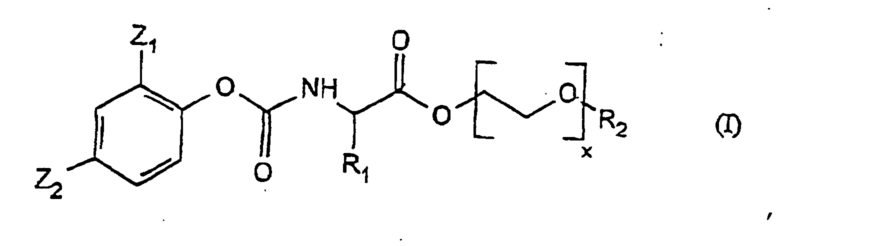 worin Z₁ und/oder Z₂ = NO2, R₁ = Phenyl, α- oder β-Indolyl, 1-Naphthyl oder 2-Naphthyl, R₂ = Me, Et, C3-C6-Alkyl oder Cycloalkyl und x ein ganze Zahl von 1 bis 5 bedeutet.

17. Reaktionsmittel nach Anspruch 9, umfassend wenigstens einen Chromophor entsprechend der allgemeinen Formel (II), 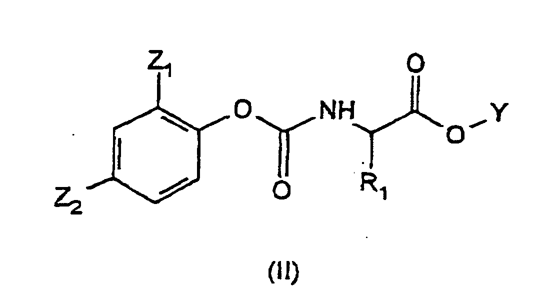 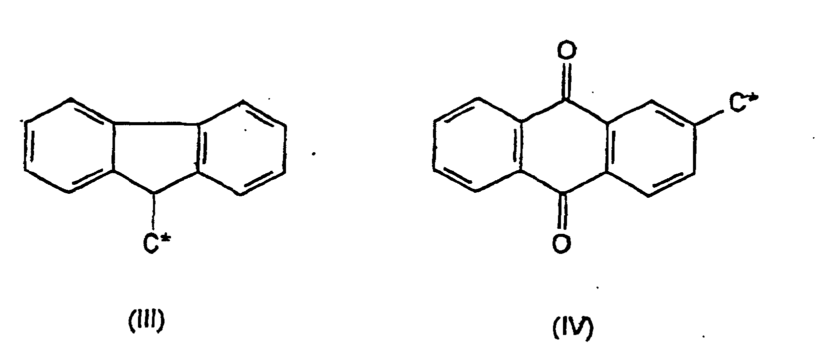 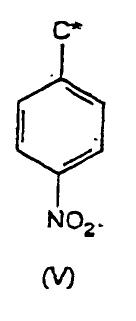 worin Z₁ und/oder Z₂ = NO2, R₁ = Phenyl, α- oder b-Indolyl, 1-Naphthyl oder 2-Naphthyl und Y einer der Formeln III bis V entspricht, wobei das Kohlenstoffatom, über das Y an den Sauerstoff der Carboxylgruppe der enantiomerenreinen Aminosäure gebunden ist, durch * markiert ist.

18. Reaktionsmittel nach Anspruch 10, entsprechend der allgemeinen Formel (VI) 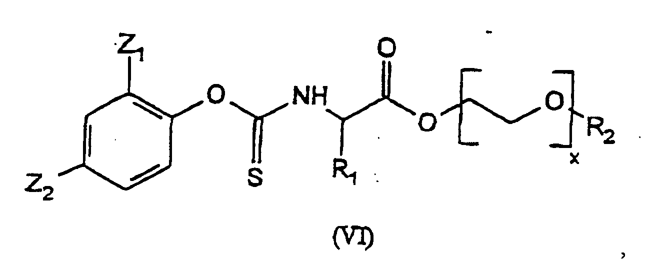 worin Z₁ und/oder Z₂ = NO2 oder F, R₁ = Phenyl, α- oder β-Indolyl, 1-Naphthyl oder 2-Naphthyl, R₂ = Me, Et, C3-C6-Alkyl oder Cycloalkyl und x ein ganze Zahl von 1 bis 5 darstellt.

19. Reaktionsmittel nach Anspruch 10, umfassend wenigstens einen Chromophor, entsprechend der allgemeinen Formel (VII) 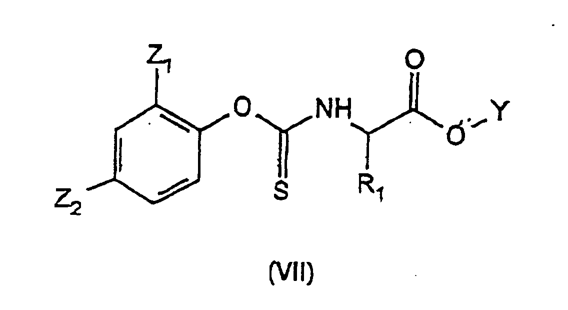 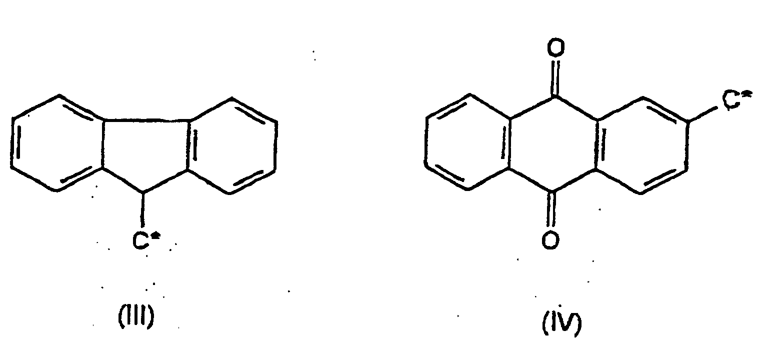 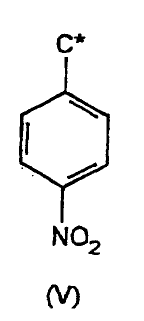 worin Z₁ und/oder Z₂ = NO2, R₁ = Phenyl, α- oder β-Indolyl, 1-Naphthyl oder 2-Naphthyl und Y einer der Formeln III bis V entspricht, wobei das Kohlenstoffatom, über das Y an den Sauerstoff der Carboxylgruppe der enantiomerenreinen Aminosäure gebunden ist, durch * markiert ist.

20. Reaktionsmittel nach einem der Ansprüche 8 bis 15, worin die enantiomerenreine Aminosäure Phenylalanin ist.

21. Reaktionsmittel nach Anspruch 20, entsprechend der Formel

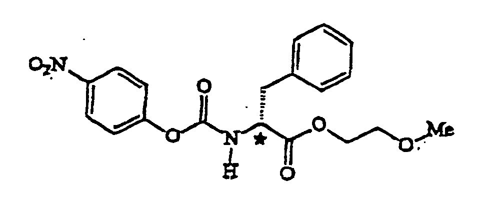

22. Lösung des Reaktionsmittels nach einem der Ansprüche 8 bis 21 in einem polaren organischen Lösungsmittel.

23. Verwendung der Lösung nach Anspruch 22 in einer automatischen Vorrichtung zur Derivatisierung und Auftrennung von Enantiomeren von organischen Verbindungen, die wenigstens ein freie funktionelle Gruppe umfassen.

24. Anwendung des Verfahrens, des Reaktionsmittels oder der Lösung nach einem der Ansprüche 1 bis 23 zur Auftrennung von Enantiomeren einer Aminosäure, eines primären oder sekundären Amins oder eines Peptids.

25. Verfahren zur Gewinnung einer enantiomerenreinen Verbindung, die wenigstens ein freie funktionelle Gruppe umfaßt, worin
(a) ein die Enantiomeren der Verbindung, die wenigstens ein freie funktionelle Gruppe aufweist, umfassendes Gemisch dem Trennverfahren nach einem der Ansprüche 1 bis 7 unterworfen wird,
(b) ein durch Auftrennen des Diastereomerengemisches erhaltenes reines Diastereomer einer Spaltung unterzogen wird,
(c) die enantiomerenreine Verbindung gewonnen wird.
